# EUROPEAN PATENT SPECIFICATION

(11) **EP 2 322 099 B1**
(45) Date of publication and mention of the grant of the patent: **11.07.2018**
(21) Application number: 09813036.2
(22) Date of filing: 03.09.2009
(51) Int. Cl.: A61B 8/00

(54) **ULTRASONOGRAPHIC DEVICE AND ULTRASONOGRAPHIC DEVICE FOCAL POINT POSITION CONTROL METHOD**
ULTRASCHALLGERÄT UND VERFAHREN ZUR STEUERUNG DES FOKUSPUNKTES DIESES ULTRASCHALLGERÄTES
DISPOSITIF ÉCHOGRAPHIQUE ET PROCÉDÉ DE COMMANDE DE POSITION DU POINT FOCAL D'UN DISPOSITIF ÉCHOGRAPHIQUE

(30) Priority: 09.09.2008 JP 2008231244
(43) Date of publication of application: 18.05.2011
(73) Proprietor: Olympus Corporation, Tokyo 192-8507 (JP)
(72) Inventor: MISONO, Kazuhiro, Hachioji-shi, Tokyo 192-8507 (JP)
(74) Representative: Gunzelmann, Rainer
(86) International application number: PCT/JP2009/065437
(87) International publication number: WO 2010/029890

(56) References cited:
- WO-A1-01/58357
- JP-A- 10 033 535
- JP-A- 11 113 899
- JP-A- 11 123 194
- JP-A- 11 290 318
- JP-A- 2001 252 276
- JP-A- 2004 209 111
- JP-A- 2007 014 485
- JP-A- 2007 014 485
- JP-A- 2007 195 892
- JP-U- 6 036 608
- US-A- 5 072 735
- US-A- 5 588 434
- US-A- 6 146 330

## Description

### Technical Field

The present invention is defined by the appended claims and relates to an ultrasound diagnostic apparatus that can change a focus position of an ultrasound image when a display region of the ultrasound image is changed, according to the changed display region, and a method for controlling a focus position of the ultrasound diagnostic apparatus. All other embodiments are merely exemplary.

### Background Art

In recent years, ultrasound diagnostic apparatuses are widely used in the medical and industrial fields. An ultrasound diagnostic apparatus repeatedly transmits ultrasound from an ultrasound transducer to a biological tissue, and receives an echo signal of ultrasound reflected from the biological tissue to display the information in a living body as a visible ultrasound tomographic image (hereafter, simply referred to as an ultrasound image).

In particular, an electronic scanning type ultrasound diagnostic apparatus, which electronically drives an ultrasound transducer to scan inside a body cavity, makes it possible to freely change the scanning method and to scan in various modes such as a flow mode including a color flow mode that can visualize the image of blood flow, besides a B-mode that displays a normal black-and-white image.

One such ultrasound diagnostic apparatus capable of scanning in various modes is, for example, an ultrasound diagnostic apparatus according to Japanese Patent Application Laid-Open Publication No. 11-290318.

The ultrasound diagnostic apparatus according to Japanese Patent Laid-Open Publication No. 11-290318 includes a transmission circuit, a mode input section, a transmission control circuit, and a transmission condition memory, wherein the transmission control circuit is configured to acquire a transmission condition corresponding to a position and a mode (a fundamental wave mode corresponding to a B-mode, or a harmonic wave mode that can display harmonic images having higher resolution than that of B-mode images) of a region of interest (hereafter referred to as an ROI) inputted from the mode input section by an operator from the transmission condition memory, and controls the transmission circuit based on the acquired transmission condition thereby adjusting operational states such as a transmission frequency, a focus depth at the time of transmission, a diameter of a transmission aperture, and the like.

Thus, the ultrasound diagnostic apparatus according to Japanese Patent Application Laid-Open Publication No. 11-290318 makes it possible to place a region of maximum sound pressure at an ROI either in a fundamental wave mode or a harmonic wave mode by setting the transmission condition such that while the focus of ultrasound is placed at the ROI in the basic wave mode, the focus of ultrasound is placed to a position slightly shallower than that of the ROI in the harmonic wave mode. As a result of that, the image quality of ROI is improved in either mode, and the change of the location of high image quality region at the time of mode switching is prevented.

Generally, in the observation by use of an ultrasound image, an operator causes a desired ultrasound tomographic image to be displayed by changing the display region of the ultrasound image to be displayed during the execution of each mode, as well as by switching each mode.

However, in the ultrasound diagnostic apparatus according to the Japanese Patent Application Laid-Open Publication No. 11-290318, the focus position is fixed even when switching of the fundamental wave mode or the harmonic wave mode is performed, and besides when such mode switching is performed, the focus position is similarly fixed even when the display region of the ultrasound image to be displayed during the execution of each mode is changed.

That is, the ultrasound diagnostic apparatus according to Japanese Patent Application Laid-Open Publication No. 11-290318 has disadvantages in that when an operator changes the display region of ultrasound image to be displayed during the execution of each mode to a desired region, a high-resolution ultrasound image cannot be displayed since a proper focus position is not achieved, and in order to achieve a proper focus position, adjustment by using an operation portion is needed, which is burdensome and inconvenient.
Document US 5,072,735 A discloses an ultrasonic imaging apparatus comprising an ultrasonic transducer for propagating ultrasonic beams to a subject, and converting echo beams reflected from the subject into an echo signal, a focus switch circuit for setting a plurality of focal depths, and a focus control circuit for outputting delay signals corresponding to the focal depths set by the focus switch circuit. The focus control circuit changes the delay signals corresponding to the preset focus depths to signals corresponding to the focus depths that are located within a display range, especially when the ultrasonic image is enlarged and shifted with at least one of the preset focus depths out of the display range.
Document WO 01/58357 A1 discloses an ultrasound diagnostic system and method for determining an acoustic output parameter of a transmitted ultrasonic beam. In one embodiment, the ultrasound system achieves a specified acoustic output parameter of a transmitted ultrasonic beam in a selected region by automatically adjusting an operating parameter of the ultrasound imaging system. In another embodiment, a region is selected in the ultrasound image that does not contain a peak acoustic output parameter of a transmitted ultrasonic beam in that region and provides an indication of the determined acoustic output parameter.
Document JP 11-1133899 A discloses an ultrasound diagnostic apparatus that sets the number and the depth of a transmission focus point according of a region of interest to be set. US 5588434 discloses an ultrasound diagnostic apparatus with a scanning range selector switch, provided to set the scanning range to a predetermined sector angle or full-circle scanning operation. Thus, the present invention has been made in view of the above-described disadvantages, and has its object to provide an ultrasound diagnostic apparatus in which a focus position of an ultrasound image is configured to be changed according to a display region when the display region of ultrasound image is changed, thereby easing the operation to achieve a proper focus position, and a method for controlling the focus position of the ultrasound diagnostic apparatus.

### Disclosure of Invention

### Means for Solving the Problem

The ultrasound diagnostic apparatus of the present invention is an ultrasound diagnostic apparatus that transmits ultrasound from an ultrasound transducer to a subject and displays an ultrasound image on a monitor, the ultrasound diagnostic apparatus comprises the features of claim 1.

The method for controlling a focus position of an ultrasound diagnostic apparatus of the present invention is a method for controlling a focus position of an ultrasound diagnostic apparatus that transmits ultrasound from an ultrasound transducer to a subject and displays an ultrasound image on a monitor, wherein the method for controlling a focus position of the ultrasound diagnostic apparatus comprises the features of claim 10.

### Brief Description of the Drawings

Fig. 1 is a block diagram showing a configuration of an ultrasound diagnostic apparatus relating to a first embodiment of the present invention;
Fig. 2 is a block diagram showing a concrete configuration of a controller of Fig. 1;
Fig. 3 is a configuration diagram showing a concrete configuration of a keyboard of Fig. 1;
Fig. 4 shows a configuration example of a touch panel operation portion of Fig. 3;
Fig. 5 is a graph showing the relationship between an ultrasound transducer number of Fig. 1 and a transmission delay amount;
Fig. 6 is mapdata showing an example of transmission timing information stored in a storage unit of Fig. 1;
Fig. 7 is a flowchart showing the flow of the control processing of a CPU to illustrate the action of the ultrasound diagnostic apparatus relating to the first embodiment;
Fig. 8 is a screen display diagram showing a state in which a focus position is displayed at the time of changing to a full circle display in a radial display;
Fig. 9 is a screen display diagram when the display range is changed from the full circle display of Fig. 8;
Fig. 10 is a screen display diagram showing a state in which the focus position is fixed when the display region is changed from the full circle display of Fig. 8 to a half circle display by use of a conventional technique;
Fig. 11 is a screen display diagram showing a state in which the focus position is changed when the display region is changed from the full circle display of Fig. 8 to a half circle display;
Fig. 12 is a flowchart showing the flow of the control processing of a CPU to illustrate the action of the ultrasound diagnostic apparatus relating to a second embodiment not forming part of the present invention;
Fig. 13 is a screen display diagram showing a state in which the focus position is moved according to the display position of an ROI during the execution of a flow mode by use of a conventional technique;
Fig. 14 is a screen display diagram showing a state in which the focus position in an ultrasound image of a B-frame is displayed regardless of the display of ROI;
Fig. 15 is a screen display diagram showing a state in which the focus position is displayed when an ROI is displayed during the execution of a flow mode;
Fig. 16 is a screen display diagram when the full circle display mode shown in Fig. 15 is changed to a half circle display mode;
Fig. 17 is a screen display diagram showing a state in which the focus position of ROI during the execution of a flow mode and the focus position of the B-mode are displayed;
Fig. 18 is a screen display diagram showing a state in which the focus position of full circle display mode is displayed when the ROI is eliminated from the state of Fig. 17;
Fig. 19 is a flowchart showing the flow of the control processing of a CPU to illustrate a variant of the ultrasound diagnostic apparatus of the second embodiment;
Fig. 20 is a screen display diagram showing the focus position of a full circle display mode during the execution of a flow mode;
Fig. 21 is a screen display diagram showing the focus position of a half circle display mode when the flow mode is executed;
Fig. 22 is a flowchart showing the flow of the control processing of a CPU to illustrate the action of an ultrasound diagnostic apparatus relating to a third embodiment not forming part of the present invention;
Fig. 23 is a screen display diagram showing a state in which an ROI before the display range is enlarged is displayed during the execution of a flow mode;
Fig. 24 is a screen display diagram showing a state in which the display region of the ROI is disposed within the display region of an ultrasound image when the display range is enlarged from the state of Fig. 23;
Fig. 25 is a flowchart showing the control processing of a CPU to illustrate the action of an ultrasound diagnostic apparatus relating to a fourth embodiment not forming part of the present invention;
Fig. 26 is a screen display diagram showing a state in which the focus position is set for each kind of connected ultrasound probes, in the display screen of an ultrasound image of a half circle display mode;
Fig. 27 is a screen display diagram showing a state in which the focus position suitable for a connected ultrasound probe is set in the display screen of an ultrasound image of a full circle display mode; and
Fig. 28 is a flowchart showing the flow of the control processing of a CPU to illustrate the action of an ultrasound diagnostic apparatus relating to a fifth embodiment not forming part of the present invention.

### Best Mode for Carrying Out the Invention

Hereafter, embodiments of the present invention will be described with reference to the drawings.

### (First Embodiment)

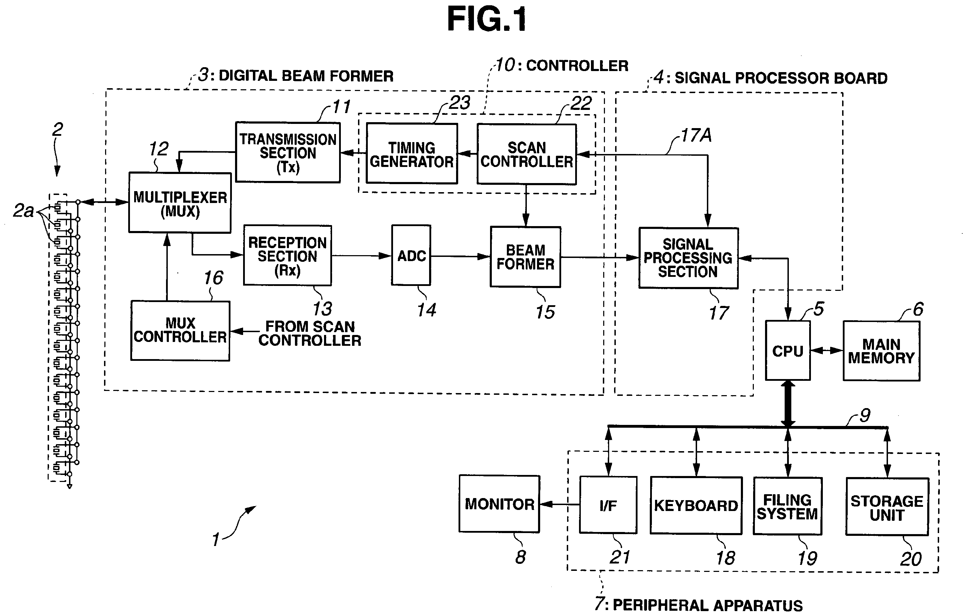

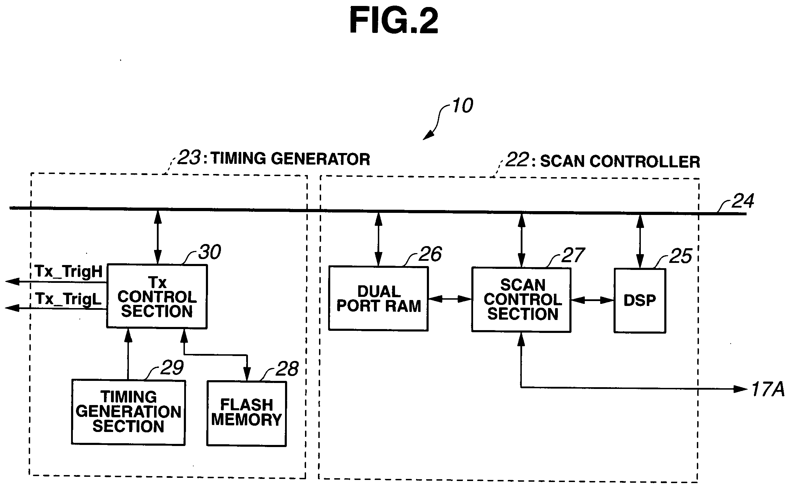

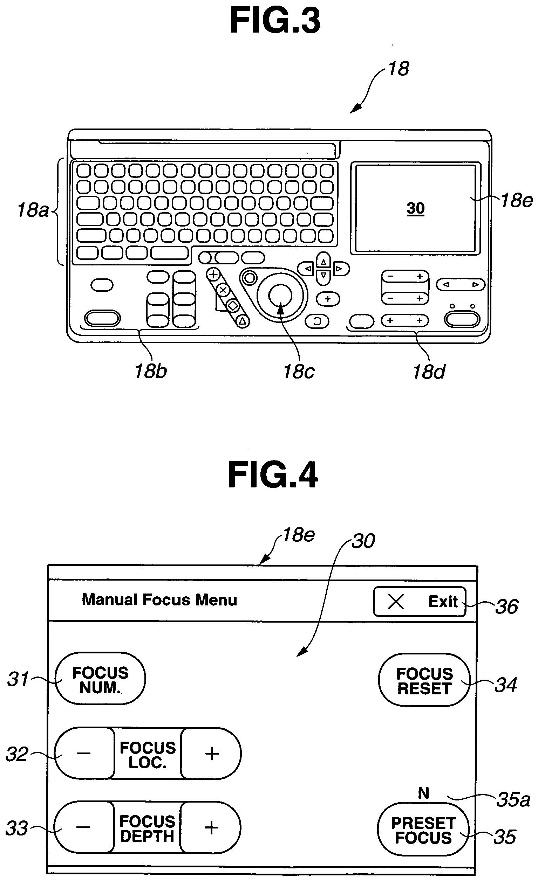

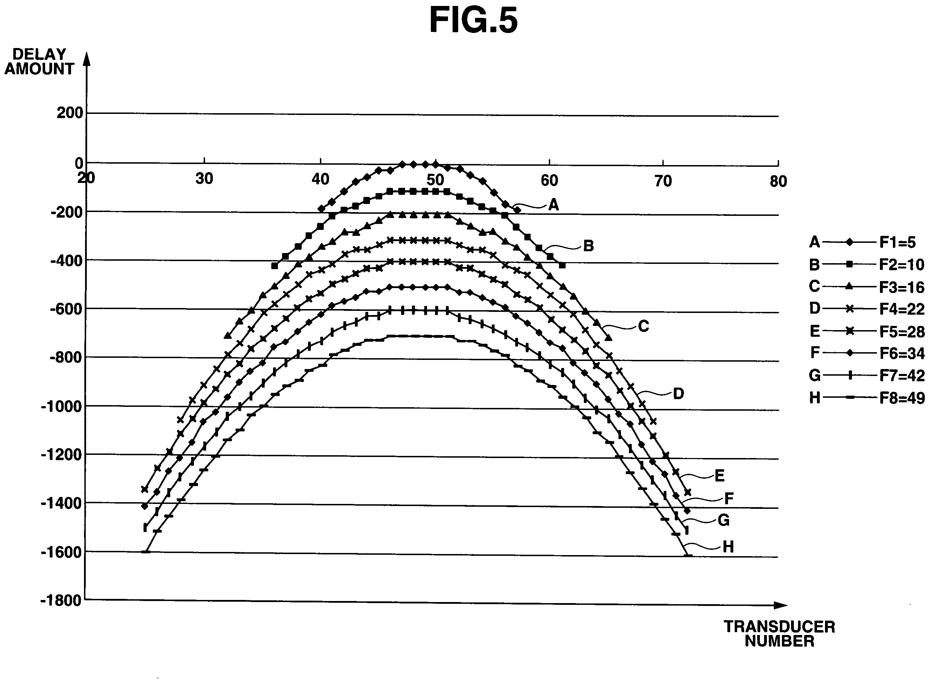

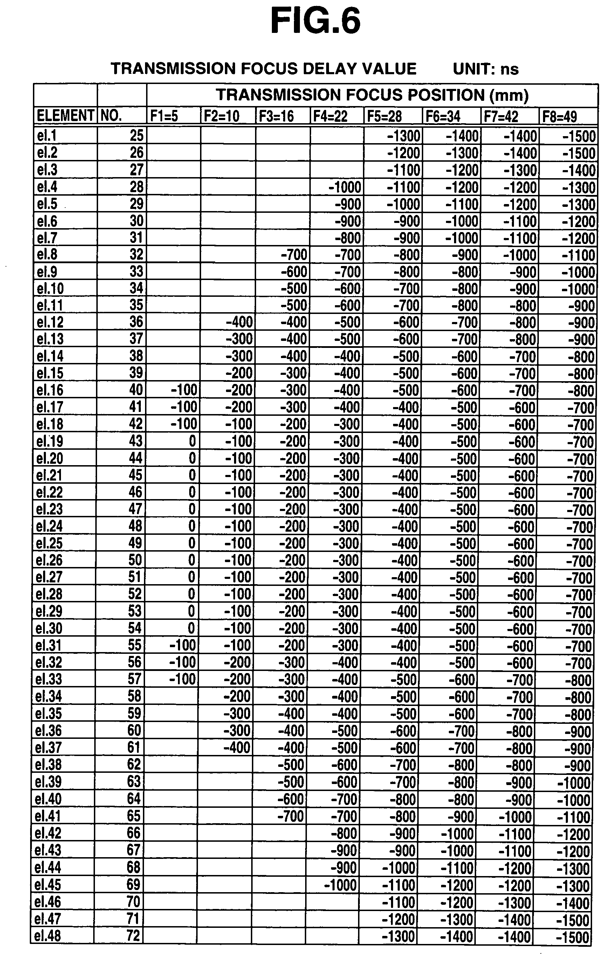

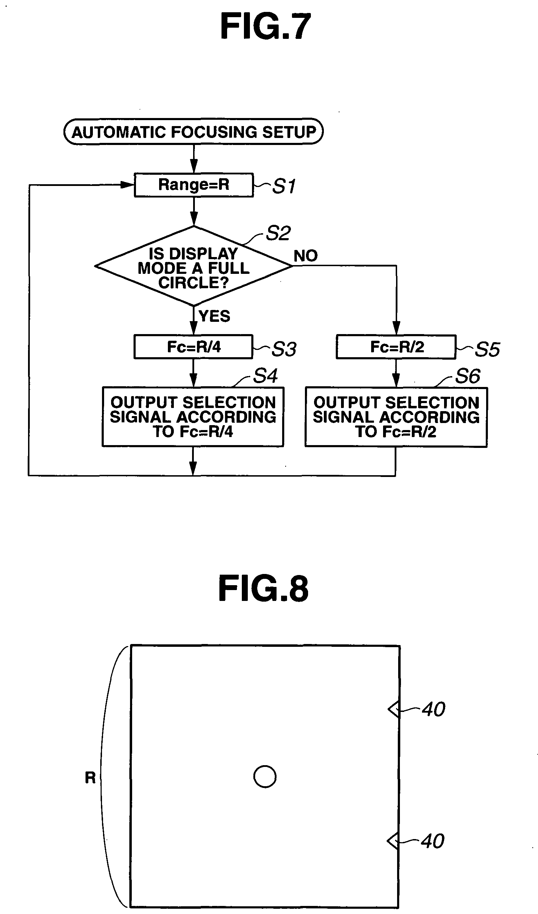

Figs. 1 to 11 relate to the first embodiment of the present invention, in which Fig. 1 is a block diagram showing a configuration of an ultrasound diagnostic apparatus; Fig. 2 is a block diagram showing a concrete configuration of a controller of Fig. 1; Fig. 3 is a configuration diagram showing a concrete configuration of a keyboard of Fig. 1; Fig. 4 is a configuration example of a touch panel operation portion of Fig. 3; Fig. 5 is a graph showing the relationship between an ultrasound transducer number of Fig. 1 and a transmission delay amount; Fig. 6 is mapdata showing an example of transmission timing information stored in a storage unit of Fig. 1; Fig. 7 is a flowchart to illustrate the action of the ultrasound diagnostic apparatus relating to the first embodiment; and Figs. 8 to 11 are screen display diagrams of a monitor for illustrating the action of the ultrasound diagnostic apparatus of the first embodiment.

As shown in Fig. 1, an ultrasound diagnostic apparatus 1 of the first embodiment is configured to include an ultrasound probe 2, a digital beam former 3, a signal processor board 4, a CPU 5, a main memory 6, a peripheral apparatus 7, and a monitor 8.

The ultrasound probe 2 is configured by arranging a plurality of ultrasound transducers 2a (hereafter, may also be referred to as "elements"). The plurality of ultrasound transducers 2a are electrically connected to the digital beam former 3 via a signal line.

It is noted that the ultrasound probe 2 to be used is of an electronic scanning type, which scans the inside of a body cavity by electronically driving the plurality of ultrasound transducers 2a. Moreover, since the specific configuration of the electronic scanning type ultrasound probe 2 is similar to that of an existing electronic scanning type ultrasound probe, the description thereof will be omitted.

The digital beam former 3 is detachably connectable with the ultrasound probe 2. This digital beam former 3 constitutes ultrasound transmission/reception means and timing adjustment means as a timing adjustment section. The digital beam former 3 and the signal processor board acquire an echo signal from the ultrasound probe 2 to generate an ultrasound image, and cause the generated ultrasound image to be displayed on the monitor 8 via a CPU 5 and a peripheral apparatus 7.

Next, the configuration of the digital beam former 3 will be described.

As shown in Fig. 1, the digital beam former 3 is configured to include a controller 10, a transmission section 11, a multiplexer (MUX) 12, a reception section 13, an ADC 14, a beam former 15, and a MUX controller 16.

The transmission section 11 as a transducer driving section constitutes transducer driving means, and the reception section 13 constitutes ultrasound reception means. The transmission section 11 generates a transmission electric signal for driving an ultrasound transducer 2a selected by the multiplexer 12, and outputs the signal to the corresponding ultrasound transducer 2a. The ultrasound transducer 2a converts the transmission electric signal supplied, with each transducer element into ultrasound, and transmits it to a subject not shown.

Then, the ultrasound reflected at the subject is converted into an electric signal again by each transducer element of the ultrasound transducer 2a, and the converted electric signal is inputted into the reception section 13 via the multiplexer 12.

The multiplexer 12 selects an ultrasound transducer 2a to be driven among the plurality of ultrasound transducers 2a of the ultrasound probe 2, based on the instruction of the MUX controller 16.

It is noted that the MUX controller 16 is configured to control the selection of the ultrasound transducer 2a by the multiplexer 12 based on the control signal from a scan controller 22 of the controller 10.

The reception section 13, which is made up of, for example, an amplifier, a BPF, a LPF, and the like, amplifies the received signal from each ultrasound transducer 2a and outputs the signal to the ADC 14.

The ADC 14 performs A/D conversion of the amplified received signal to convert the signal into digital data and outputs the same to the beam former 15.

The beam former 15 delays and combines each received data which is digitized according to the driving of the plurality of ultrasound transducers 2a based on a timing signal from a scan controller 22 of the controller 10, and outputs the combined data to a signal processing section 17 in the signal processor board 4.

On the other hand, when ultrasound is transmitted from an ultrasound transducer 2a of the ultrasound probe 2, the controller 10, which is transmission adjustment means, adjusts the transmission timing of the ultrasound by the transmission section 11.

The controller 10 is configured to include the scan controller 22 and a timing generator 23.

The scan controller 22 is electrically connected to the signal processing section 17 of the signal processor board 4 by means of a bus 17A, and is controlled by the CPU 5 to be described below. The scan controller 22 controls the timing generator 23 for the transmission section based on the instruction of the CPU 5 so that the transmission timing of ultrasound by the transmission section 11 is controlled. Moreover, the scan controller 22 generates a timing signal and outputs the same to the beam former 15.

It is noted that concrete configurations of the controller 10 that constitutes a principal part of the present invention will be described later.

Next, the configurations of the signal processor board 4, the CPU 5, the main memory 6, and the peripheral apparatus 7 will be described.

The signal processor board 4 is configured to include a signal processing section 17 to which combined data from the digital beam former 3 is inputted. It is noted that the signal processor board 4 may be configured by appropriately providing necessary blocks without being limited to the configuration shown in Fig. 1.

The signal processing section 17 performs signal processing according to the kinds of combined data to be inputted.

For example, in the case of B-mode, the signal processing section 17 performs processing for generating B-mode data, such as band pass filtering, Log compression, wave detection, gain adjustment, contrast adjustment, and the like. Moreover, in the case of a flow mode, the signal processing section 17 performs processing for generating color data relating to blood flow. Then, the signal processing section 17 stores processed sound ray data in a frame memory not shown for every one frame.

The CPU 5 performs coordinate transformation and image processing on the sound ray data stored in a frame memory not shown of the signal processing section 17 to generate digital ultrasound data for image display and to output the same to an I/F 21 of the peripheral apparatus 7 via an internal bus 9.

The I/F 21 includes a video processing section and converts digital ultrasound data into an analog image signal by using the video processing section to output the same to the monitor 8 so that an ultrasound image base on the analog image signal is displayed on the monitor 8.

The peripheral apparatus 7 is configured to include the I/F 21, a keyboard 18, a filing system 19, and a storage unit 20 that constitutes storage means.

The filing system 19 stores various data necessary for performing diagnosis by use of the ultrasound diagnostic apparatus 1, for example, patient information and ultrasound image data for each patient, etc. It is noted that the filing system 19 may be configured to obtain necessary data and information by connecting to a server via a network laid in, for example, a hospital.

The keyboard 18 as an operation portion constitutes operation means, and is configured, for example as shown in Fig. 3, to include various key operation portion 18a, a first switch operation portion 18b made up of a plurality of switches, a second switch operation portion 18c made up of a trackball and the like, a third switch operation portion 18d made up of a plurality of switches for increasing/decreasing various settings, and a fourth switch operation portion 18e which is a touch panel operation portion by use of an LCD.

It is noted that the keyboard 18 is not limited to the configuration shown in Fig. 3, and may be configured such that the number of the switch operation portions is appropriately increased or decreased, and the layout of the switch operation portion is changed.

Moreover, the fourth switch operation portion 18e displays, for example, an operation panel screen 30 for manual focusing menus, and there are arranged in the operation panel screen 30, a first button 31 for selecting and instructing the number of focusing stages, a second button 32 for adjusting focusing location, a third button 33 for adjusting focusing depth, a fourth button 34 for resetting focusing, a fifth button 35 for presetting focusing, and a sixth button 36 for cancelling the mode for the manual focusing menu. It is noted that a focusing location (focus position) display section 35a is provided in the vicinity of the fifth button 35, and a focus position that is set to any one of a near point position (N), a medium point position (M), and a far point position (F) is displayed in the focusing location display section 35a.

The storage unit 20 stores a program that is necessary for controlling the entire ultrasound diagnostic apparatus 1 such as the digital beam former 3, the signal processor board 4, and the peripheral apparatus 7, by the CPU 5.

Further, the storage unit 20 stores user setup information, such as transmission timing information according to the display region to be displayed on the monitor 8, and initial setup information of focus position according to the kind of the ultrasound probe 2, in an ultrasound image.

Next, a concrete configuration of the controller 10 that constitutes a principal part of the present invention, and transmission timing information that is supplied to the controller 10 through the control by the CPU 5 will be described by using Figs. 2, 5, and 6.

As shown in Fig. 2, the controller 10, which constitutes display region changing means as a display region changing section together with the CPU 5, is configured to include a scan controller 22 and a timing generator 23, which are electrically connected by an internal bus 24.

The scan controller 22 is configured to include a DSP (Digital Signal Processor) 25, a dual port RAM 26, and a scan control section 27. The scan control section 27, which is connected to the bus 17A, is controlled by the CPU 5.

The scan control section 27 controls the timing generator 23 according to the instruction from the CPU 5.

The timing generator 23 is configured to include a flash memory 28, a timing generation section 29, and a Tx control section 30.

The Tx control section 30 outputs to the transmission section 11 a control signal for changing the transmission timing of ultrasound by the transmission section 11 based on the timing signal from a timing generation section 29, according to the instruction from the scan control section 27.

Moreover, in the present embodiment, when transmission timing information is supplied from the scan controller 22, the Tx control section 30 writes the transmission timing information into the flash memory 28, and outputs a timing signal according to the transmission timing information based on a selection signal from the CPU 5, out of the transmission timing information, to the transmission section 11.

It is noted that the timing signal from the Tx control section 30 desirably outputs triggers of two types to generate, for example, a bipolar pulse of a positive pulse and a negative pulse.

Therefore, as the result of such timing signal being outputted to the transmission section 11, the transmission section 11 changes the transmission timing of ultrasound to be transmitted by the ultrasound transducer 2a, based on the transmission timing. Thus, the focus position of an ultrasound image is changed.

In this case, to change the focus position of an ultrasound image, it is necessary to adjust a delay amount according to the sequence position of the ultrasound transducer 2a (ultrasound transducer number).

Fig. 5 shows the relationship between the ultrasound transducer number (corresponding to the sequence position) according to a plurality of focus positions A to H (in Fig. 5, focus positions F1 to F8 are designated by A to H) and the delay amount.

It is noted that the ultrasound diagnostic apparatus 1 of the present embodiment is configured to vary the transmission aperture depending on the focus position as shown in Fig. 5. Moreover, the ultrasound diagnostic apparatus 1 can vary the transmission aperture, that is, the number of the ultrasound transducers 2a to be driven, according to the B-mode or the flow mode. Further, the ultrasound diagnostic apparatus 1 switches the number of the ultrasound transducers 2a to be used at the deepest point of the focus position according to the ultrasound probe 2 to be used.

Moreover, mapdata of transmission focusing delay data showing such delay amount is shown in Fig. 6. That is, mapdata of transmission focusing delay data as shown in Fig. 6 is stored in the storage unit 20 as the timing information for each kind of the ultrasound probe 2.

In the present embodiment, the CPU5 as a control section constitutes control means, and changes the focus position of an ultrasound image by controlling the transmission timing of the timing generator 23 in response to a change of the display region to be displayed on the monitor 8.

That is, when there is a change of the display region by an operation of the keyboard 18, the CPU 5 reads out the mapdata of the transmission focusing delay data shown in Fig. 6 from the storage unit 20, and writes the data into the flash memory 28 of the timing generator 23 via the scan controller 22.

Then, the CPU 5 recognizes the display region after the change, and outputs a selection signal for selecting transmission focusing delay data corresponding to the display region after the change to the Tx control section 30 via the internal bus 24.

The Tx control section 30 extracts the transmission focusing delay data based on the selection signal from the mapdata written in the flash memory 28, and outputs a timing signal based on the transmission focusing delay data to the transmission section 11 thereby changing the focus position.

Next, the action of the ultrasound diagnostic apparatus of the first embodiment will be described with reference to Figs. 7 to 11.

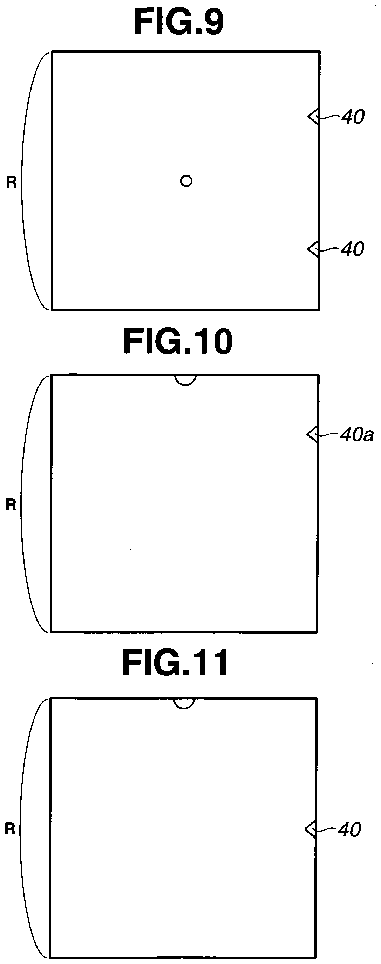

It is noted that Fig. 7 is a flowchart showing the flow of the control processing of a CPU; Fig. 8 is a screen display diagram showing a state in which a focus position is displayed at the time of changing to a full circle display in a radial display; Fig. 9 is a screen display diagram when the display range is changed from the full circle display of Fig. 8; Fig. 10 is a screen display diagram showing a state in which the focus position is fixed when the display region is changed from the full circle display of Fig. 8 to a half circle display by use of a conventional technique; and Fig. 11 is a screen display diagram showing a state in which the focus position is changed when the display region is changed from the full circle display of Fig. 8 to a half circle display.

Here, suppose that the operator performs automatic focusing setup of ultrasound image displayed in the monitor 8 by use of the ultrasound diagnostic apparatus 1 shown in Fig. 1. In this case, the CPU 5 of Fig. 1 reads out a program shown in Fig. 7 and stored in the storage unit 20.

The CPU 5 determines the display range (indicated by R: Range in Fig. 7) in the currently executed mode in the processing of step S1 and moves to judgment processing in the following step S2.

In the judgment processing in step S2, judging whether or not the currently executed display mode is a full circle display mode in a radial display, the CPU 5 moves the process to step S3 when the display mode is a full circle display mode, and moves the process to step S5 when the display mode is not a full circle display mode.

Since the processing of step S3 is a case in which the display mode is a full circle display mode, the CPU 5 calculates and sets the focus position (indicated by Focusing point: Fc in Fig. 7) Fc at R/4 or in the vicinity thereof, thereafter moving the process to step S4.

In the processing of step S4, the CPU 5 generates a selection signal that disposes the focus position Fc at R/4 or in the vicinity thereof, and outputs the signal to the timing generator 23 via the scan controller 22 in the controller 10.

It is noted that when automatic focusing setup is executed, the CPU 5 reads out the mapdata of the transmission focusing delay data shown in Fig. 6 from the storage unit 20, and writes the data into the flash memory 28 of the timing generator 23 via the scan controller 22.

Then, the Tx control section 30 of the timing generator 23 (see Fig. 2) extracts transmission focusing delay data based on the supplied selection signal from the mapdata written into the flash memory 28, and outputs a timing signal based on the transmission focusing delay data to the transmission section 11, thereby causing the focus position of the ultrasound image displayed on the monitor 8 to be changed.

It is noted that in this case, the CPU 5 causes a video signal of the marker 40 indicating a focus position to be displayed as being superposed on the ultrasound image data being displayed by using the signal processing section 17 and the I/F 21.

Therefore, performing these processing will make it possible to display an ultrasound image of a full circle display, in which the marker 40 indicating the focus position is disposed at two points along one side of the display frame (at positions at 1/4 of the display range R or in the vicinity thereof), and the focus position is located at the position of the marker 40.

In this case, since in a full circle display mode, the display image is a radial display image of 360 degrees, two more markers 40 in addition to the above-described marker 40 may be provided at a position at 1/4 of display range R or in the vicinity thereof along a upper or lower side of the image display frame of the monitor 8.

Moreover, when it is supposed that the display range R shown in Fig. 8 is, for example, a 6 cm range, the radius of the ultrasound image in a full circle display will be 3 cm, and therefore the marker 40 in this case will be disposed at each position of 1.5 cm, which is a half of the radius or in the vicinity thereof.

In this case, even if the display range R is changed to, for example, 12 cm as shown in Fig. 9, the radius of ultrasound image being displayed in a full circle display will be 6 cm, and the marker 40 will be disposed at each position of 3 cm, which is a half of the radius or in the vicinity thereof.

On the other hand, when the display mode is judged not to be a full circle display mode in the judgment processing of the step S2, the CPU 5 recognizes that the display region has been changed from the full circle display mode to a half circle display mode, and calculates and sets the focus position Fc at R/2 or in the vicinity thereof in the processing of step S5, thereafter moving the process to step S6.

In the processing of step S6, the CPU 5 generates a selection signal that disposes the focus position Fc at R/2 or in the vicinity thereof, and outputs the signal to the timing generator 23 via the scan controller 22 in the controller 10.

Then, the Tx control section 30 of the timing generator 23 extracts transmission focusing delay data based on the supplied selection signal from the mapdata written in the flash memory 28, and outputs the timing signal based on the transmission focusing delay data to the transmission section 11, thereby causing the focus position of the ultrasound image displayed on the monitor 8 to be changed.

That is, in conventional art, even if the display region is changed from a full circle display mode to a half circle display mode, for example, in the display range R (R = 12 cm) shown in Fig. 8, the marker 40a indicating a focus position will be disposed at a center position of the set display range, that is, at a position of 1.5 cm without change from the full circle display mode, as shown in Fig. 10.

However, in the present embodiment, performing the above-described processing makes it possible that even if the display region is changed from a full circle display mode having a display range of 12 cm shown in Fig. 9 to a half circle display mode, the marker 40 indicating a focus position is disposed at a position of 1/2 of the display range R (in this case, 6 cm since it is a half circle mode)(3 cm) or in the vicinity thereof as shown in Fig. 11, and an ultrasound image in a half circle display, the focus position of which is located at the position of the marker 40, is displayed.

Then, after executing the processing of step S4 or step S6, the CPU 5 returns the process to step S1, thereby providing for any change of the display region (display range) of the monitor 8 in an ultrasound image.

Therefore, according to the first embodiment, the focus position of the ultrasound image may be configured to be changed according to a display region when the display region of an ultrasound image is changed, thereby easing the operation to achieve a proper focus position.

Moreover, since an ultrasound image in which a focus position has been located is displayed on the monitor 8 even when the display region is changed, it becomes possible to present the operator with easier-to-see and a higher resolution ultrasound images thereby contributing to the improvement of observation performance by an ultrasound diagnostic apparatus.

It is noted that in the first embodiment, although it has been described that two of the markers 40 indicating a focus position are disposed on one vertical side of the image display frame of the monitor 8, this configuration is not restrictive, and for example, configuration may be such that more markers 40 are disposed on one horizontal side of the image display frame of the monitor 8.

### (Second Embodiment)

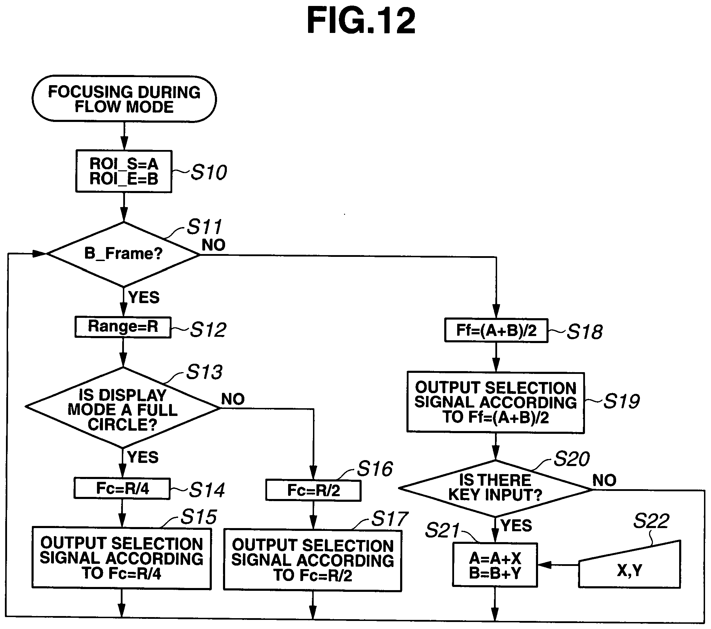

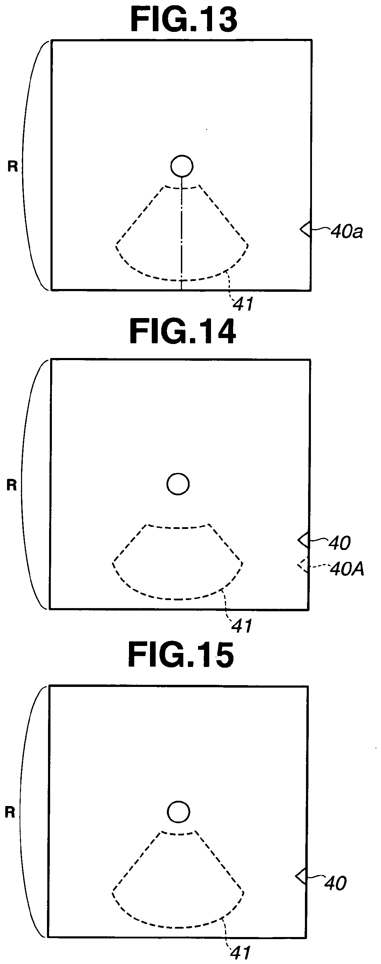

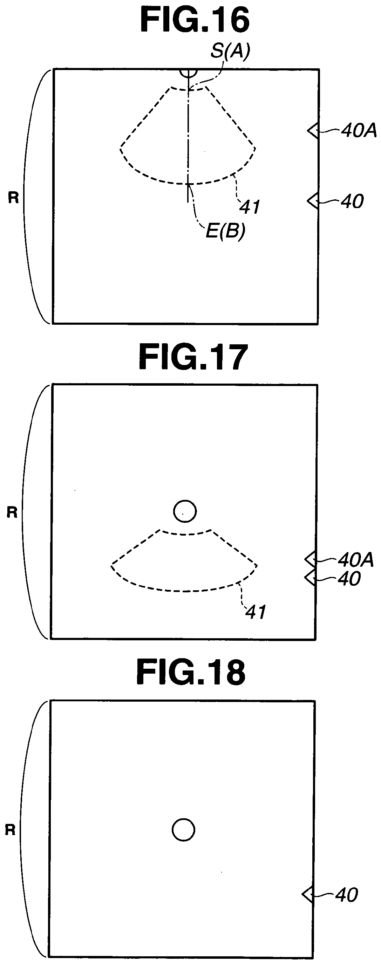

Figs 12 to 18 relate to a second embodiment not forming part of the present invention, in which Fig. 12 is a flowchart showing the flow of the control processing of a CPU to illustrate the action of the ultrasound diagnostic apparatus of the second embodiment; Fig. 13 is a screen display diagram showing a state in which the focus position is moved in response to the display position of ROI when a flow mode is executed by use of a conventional technique; Fig. 14 is a screen display diagram showing a state in which the focus position in an ultrasound image of a B-frame is displayed regardless of the display of ROI; Fig. 15 is a screen display diagram showing a state in which the focus position is displayed when an ROI is displayed during the execution of a flow mode; Fig. 16 is a screen display diagram when the full circle display mode shown in Fig. 15 is changed to a half circle display mode; Fig. 17 is a screen display diagram showing a state in which the focus position of ROI during the execution of a flow mode and the focus position of the B-mode are displayed; and Fig. 18 is a screen display diagram showing a state in which the focus position of the full circle display mode is displayed when ROI is eliminated from the state of Fig. 17.

An ultrasound diagnostic apparatus 1 of the second embodiment is similarly configured as the ultrasound diagnostic apparatus 1 of the first embodiment, but differs in the control operation of the CPU 5 constituting control means. It is noted that hereafter, the control operation that differs from that of the first embodiment will be described.

To be specific, the ultrasound diagnostic apparatus 1 of the second embodiment is configured such that the change of the display region of the monitor 8 corresponds to the change of the display region of an ROI that is displayed during the execution of a flow mode, and the focus position of an ultrasound image is changed according to the display region of the ROI even when the display region of the ROI is changed.

Moreover, the ultrasound diagnostic apparatus 1 is configured such that each image of a B-frame and a flow frame that are obtained by time division of a B-mode and a flow mode are displayed in superposition on the monitor 8 during the execution of a flow mode, and the focus position of ultrasound image of the B-frame and the focus position of ultrasound image of the flow frame are changed respectively during the execution of the flow mode.

Next, the operation of the ultrasound diagnostic apparatus of the second embodiment will be described by using Figs. 12 to 16.

Here, suppose that the operator performs the automatic focusing setup of the ultrasound image displayed on the monitor 8 during the execution of a flow mode by using the ultrasound diagnostic apparatus 1 shown in Fig. 1. In this case, the CPU 5 of Fig. 1 reads out and executes a program corresponding to the flow mode shown in Fig. 12 and stored in the storage unit 20.

The CPU 5 sets the start coordinate (indicated by S in Figs. 12 and 16) of an ROI 41, ROI_S, to "A", and the end coordinate (indicated by E in Figs. 12 and 16) of the ROI, ROI_E, to "B" in the processing of step S10, and moves the process to step S11.

In the processing of step S11, the CPU 5 judges whether or not ultrasound data to be image-processed is B-frame data, and when the data is B-frame data, the CPU 5 executes routines from step S12, and otherwise moves the process to step S18.

When the data is ultrasound data of B-frame, as with the first embodiment, the CPU 5 determines and sets the current display range R in the processing of step S12, and moves to the judgment processing of the following step S13.

In the judgment processing of step S13, the CPU 5 judges whether or not the currently executed display mode is a full circle display mode in a radial display, and when the display mode is a full circle display mode, the CPU 5 moves the process to step S14, and when the display mode is not a full circle display mode, moves the process to step S16.

When the display mode is the full circle display mode, the CPU 5 calculates and sets the focus position Fc at R/4 or in the vicinity thereof in the processing of step S14 as with the processing of step S3 and step S4 (see Fig. 7) in the first embodiment. Next, the CPU 5 generates a selection signal that disposes the focus position Fc at R/4 or in the vicinity thereof, and outputs the selection signal to the timing generator 23 via the scan controller 22 in the controller 10 in the following step S15.

Then, the Tx control section 30 of the timing generator 23 (see Fig. 2) extracts transmission focusing delay data based on the supplied selection signal from the mapdata written in the flash memory 28, and outputs a timing signal based on the transmission focusing delay data to the transmission section 11, thereby causing the focus position of the ultrasound image displayed on the monitor 8 to be changed.

Therefore, when the ultrasound data is B-frame data and the display mode is a full circle mode during the execution of a flow mode, it is possible to display on the monitor 8 an ultrasound image in a full circle display in which a marker 40 that indicates a focus position located at 1/4 of the display range R or in the vicinity thereof is disposed, and the focus position is located at the position of the marker 40, as with the first embodiment.

That is, although in conventional arts, a marker 40a indicting a focus position is changed in synchronization with the display position of an ROI during the execution of a flow mode as shown in, for example, Fig. 13; in the present embodiment, when the ultrasound data is B-frame data during the execution of a flow mode, the marker 40 is disposed at a position of 1/4 of the display range R or in the vicinity thereof regardless of the display position of the ROI 41 similarly to the first embodiment, as shown in Fig. 14.

On the other hand, when the display mode is a half circle display mode, the CPU 5 calculates and sets the focus position Fc at R/2 or in the vicinity thereof in the processing of step S16 as with the processing of step S5 and step S6 in the first embodiment (see Fig. 7). Next, the CPU 5 generates a selection signal that disposes the focus position Fc at R/2 or in the vicinity thereof in the processing of step S17, and outputs the selection signal to the timing generator 23 via the scan controller 22 in the controller 10.

Then, the Tx control section 30 of the timing generator 23 (see Fig. 2) extracts transmission focusing delay data based on the supplied selection signal from the mapdata written in the flash memory 28, and outputs a timing signal based on the transmission delay data to the transmission section 11, thereby causing the focus position of the ultrasound image displayed on the monitor 8 to be changed.

Therefore, when the ultrasound data is B-frame data and the display mode is a half circle mode during the execution of a flow mode, it is possible to display on the monitor 8 an ultrasound image in half circle display in which a marker 40 that indicates a focus position located at 1/2 of the display range R or in the vicinity thereof is disposed as shown in Fig. 16, and the focus position is located at the position of the marker 40, as with the first embodiment.

Next, when the ultrasound data is judged not to be B-frame data in the judgment processing of the above-described step S11, the CPU 5 judges that the ultrasound data is flow frame data for executing a color flow mode to display an ROI 41, and calculates and sets the focus position Ff of the ROI 41 at (A+B)/2 in the processing of step S18.

Next, the CPU 5 generates a selection signal that disposes the focus position Ff at (A+B)/2 or in the vicinity thereof, and outputs the signal to the timing generator 23 via the scan controller 22 in the controller 10.

Then, the Tx control section 30 of the timing generator 23 (see Fig. 2) extracts transmission focusing delay data based on the supplied selection signal from the mapdata written in the flash memory 28, and outputs a timing signal based on the transmission focusing delay data to the transmission section 11, thereby causing the focus position of the ROI 41 displayed on the monitor 8 to be changed.

Therefore, when the ultrasound data is flow frame data during the execution of a flow mode, it is possible to display on the monitor 8 a blood flow image of an ROI 41 in which a marker 40A indicating a focus position is disposed at a position of (A+B)/2 or in the vicinity thereof in the display region of an ROI 41, and the focus position is located at the position of the marker 40A, as shown in Fig. 14.

In the second embodiment, even when the operator changes the display region of an ROI 41, the operator can change the focus position of the ROI 41 without performing burdensome operation.

That is, the CPU 5 judges if there is a key input by the key operation of the keyboard 18 for changing the display region of the ROI 41 in the judgment processing of the following step S20, and when there is a key input, the CPU 5 moves the process to step S21, and when there is no key input, returns the process to step S11.

In the processing of step S21, the CPU 5 takes in changed coordinates (X, Y) of an ROI 41 based on the key input of the operator that is detected in step S22, and sets the start coordinate A of the ROI 41 to (A + X) and the end coordinate B to (B + Y) to change the display region of the ROI 41.

In this case, as the result of the CPU 5 returning the process to step S11 and performing the processing of step S18 and step S19 described above again, even if the display region of the ROI 41 is changed, the marker 40A indicating the focus position is disposed approximately at the center of the ROI 41 after the change (see Figs. 14 and 16).

It is noted that in the second embodiment, when an ROI 41 is displayed during the execution of a flow mode, the CPU 5 controls the focus position not to be changed for the marker 40 indicating the focus position in an ultrasound image of B-frame even if the display mode is changed to a full circle display mode as shown in Fig. 15 or a half circle display mode (convex display) as shown in Fig. 16.

Further, in the second embodiment, when operation to eliminate the display of an ROI 41 is performed during the execution of a flow mode, the CPU 5 performs the control to eliminate the display of the ROI 41, as well as performs control such that only the marker 40 indicating the focus position in the ultrasound image of B-frame is displayed.

Therefore, although in such a case, the marker 40A is disposed approximately at the center of the ROI 41, and the marker 40 is disposed at the focus position (position at 1/4 of the display range R or in the vicinity thereof) in the ultrasound image of B-frame, on the monitor 8 as shown in Fig. 17, when the ROI 41 is eliminated, only the marker 40 is disposed at the focus position (position at 1/4 of the display range R or in the vicinity thereof) in the ultrasound image of B-frame as shown in Fig. 18.

It is noted that in the second embodiment, although it has been described that both of the marker 40A indicating the focus position of the ROI 41 and the marker 40 indicating the focus position of an ultrasound image of B-frame are displayed in superposition on one screen, it may be configured such that only the marker 40 indicating the focus position of B-mode is displayed. Since the focus position of flow mode is located around the center of an ROI, it is possible for a user to keep track of the focus position even if the display thereof is omitted.

Therefore, according to the second embodiment, it becomes possible to change the focus position of B-frame and the focus position of flow frame respectively during execution of a flow mode, and further it becomes possible to dispose the marker 40A such that the focus position always coincides with the approximate center position of the ROI 41 even when the display region of the ROI 41 is changed.

As a result of this, even during the execution of a flow mode, it becomes possible to ease the operation to achieve a proper focus position. Other advantages are similar to those of the first embodiment.

### (Variant)

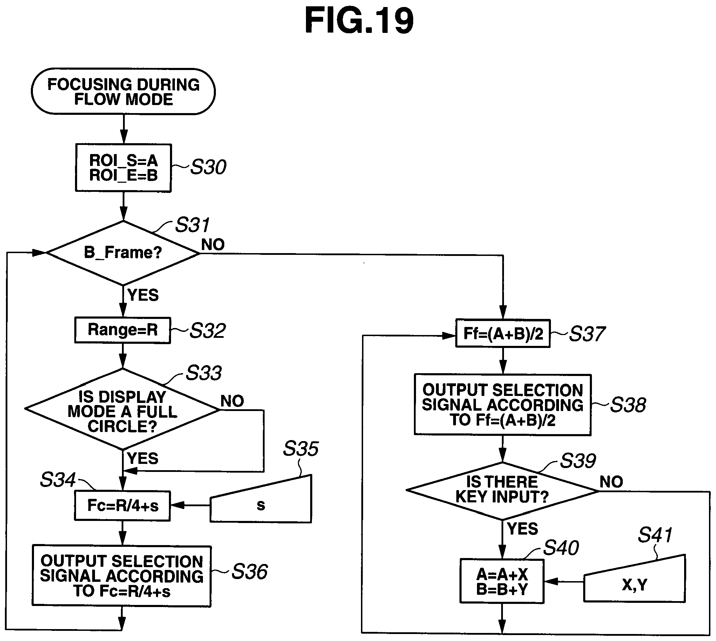

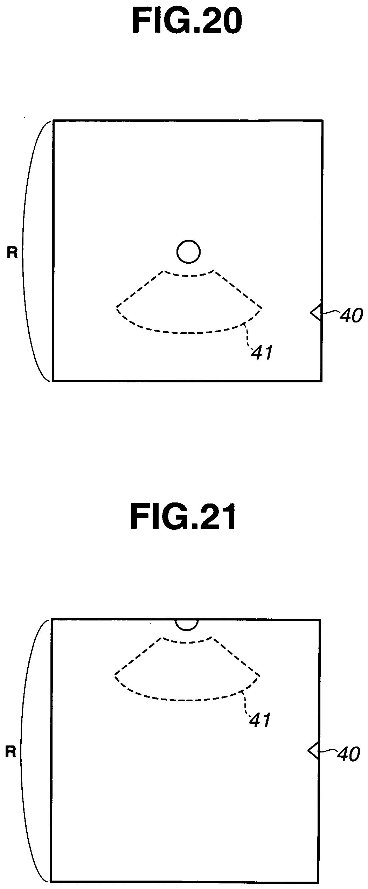

Figs. 19 to 21 show a variant of the ultrasound diagnostic apparatus of the second embodiment, in which Fig. 19 is a flowchart showing the flow of the control processing of a CPU; Fig. 20 is a screen display diagram showing the focus position of a full circle display mode during the execution of a flow mode; and Fig. 21 is a screen display diagram showing the focus position of a half circle display mode during the execution of a flow mode.

In the present variant, although the CPU 5 performs control in an approximately similar manner as in the second embodiment, the CPU 5 can change the focus position of an ultrasound image to a position at 1/4 of the display range R or in the vicinity thereof when the ultrasound data is B-frame data during the execution of a flow mode, even if the display mode is a full circle display mode or a half circle display mode, and further can perform control such that the focus position can be freely changed according to a key operation by an operator.

To be specific, although the program (step S30 to step S41) shown in Fig. 19 and to be executed by the CPU 5 is approximately the same as that in the second embodiment (see Fig. 12), the judgment processing in step S33, and the processing in step S35 and step S36 are different.

That is, the CPU 5 moves the process to step S34 which is similar to the case in which the display mode is a full circle display mode, even if the display mode is not a full circle mode, that is, the display mode is a half circle mode in the processing of step S33.

In the processing of step S34, the CPU 5 takes in an amount of movement S of the focus position Fc based on a key input by an operator detected in step S35, and calculates and sets the focus position Fc at R/4 + S, thereafter moving the process to step S36.

In the processing of step S36, the CPU 5 generates a selection signal that disposes the focus position Fc at R/4 + S, and outputs the signal to the timing generator 23 via the scan controller 22 in the controller 10.

Then, the Tx control section 30 of the timing generator 23 extracts transmission focusing delay data based on the supplied selection signal from the mapdata written in the flash memory 28, and outputs a timing signal based on the transmission focusing delay data to the transmission section 11, thereby causing the focus position of the ultrasound image displayed on the monitor 8 to be changed.

By performing such processing, it becomes possible to dispose the focus position of an ultrasound image of B-frame during the execution of a flow mode always at the same focus position regardless of whether the display mode is a full circle display mode or a half circle display mode as shown in Fig. 20 and Fig. 21, and also to change the focus position to a focus position based on the operation to change focus position by the operator.

It is noted that similar operation as in the second embodiment is performed on the focus position of an ROI 41 in the case of the flow frame during the execution of a flow mode, and a marker 40A is disposed at a similar focus position.

Therefore, according to the present variant, since the marker 40 can be disposed always at the same focus position regardless of the types of display mode when the ultrasound data is B-frame data during the execution of a flow mode, it is possible to make it easy for the operator to change and adjust the focus position with reference to the marker 40. Other advantages are the same as those of the second embodiment.

### (Third Embodiment)

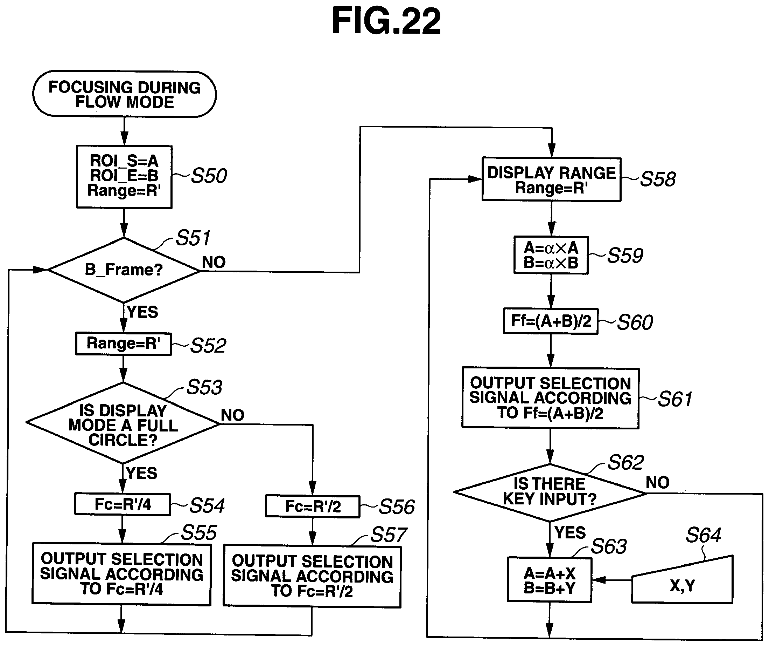

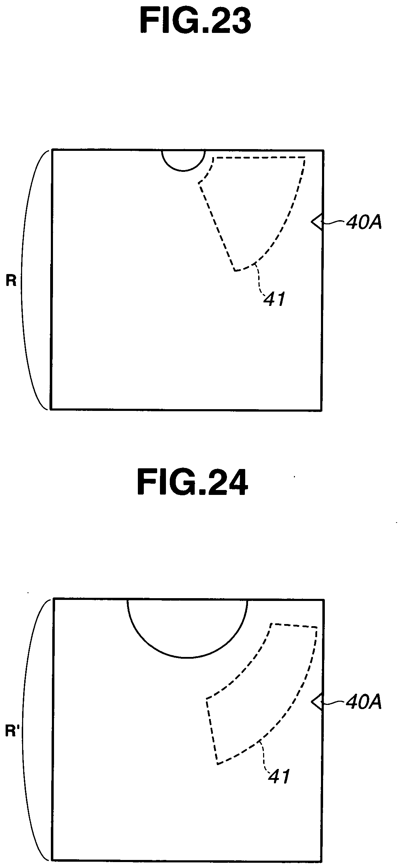

Figs. 22 to 24 relate to a third embodiment not forming part of the present invention, in which Fig. 22 is a flowchart showing the flow of the control processing of a CPU to illustrate the action of an ultrasound diagnostic apparatus relating to the third embodiment; Fig. 23 is a screen display diagram showing a state in which an ROI before the display range is enlarged during the execution of a flow mode is displayed; Fig. 24 is a screen display diagram showing a state in which the display region of the ROI is disposed within the display region of an ultrasound image when the display range is enlarged from the state of Fig. 23.

An ultrasound diagnostic apparatus 1 of the third embodiment is similarly configured as the ultrasound diagnostic apparatus 1 of the first embodiment, but differs in the control operation of the CPU 5 constituting control means. It is noted that hereafter, the control operation that differs from that of the first embodiment will be described.

In the third embodiment, although the CPU 5 performs control in approximately the same way as in the second embodiment, when the display region (display range R) displayed on the monitor 8 in the ultrasound image during the execution of a flow mode is changed, the CPU 5 controls the size of an ROI 41 such that the ROI 41 is disposed within the display region of the ultrasound image.

To be specific, the program (step S50 to step S64) that is executed by the CPU 5 and shown in Fig. 22 is approximately the same as the program (see Fig. 12) in the second embodiment, but the program differs in the processing of step S50, and is newly added with the processing of steps S58 and S59.

Here, suppose that as shown in Fig. 23, the display range R has been enlarged by the operation of the keyboard 18 by the operator during the execution of a flow mode from a state in which an ultrasound image and an ROI 41 corresponding to the display range R are displayed on the monitor 8.

The CPU 5 executes the program to be executed after the enlargement of the display range R shown in Fig. 22, setting the ROI 41 start coordinate ROI_S to "A" and the ROI end coordinate ROI_E to "B" in the processing of step S50, and further determines the enlarged display range R' thereafter moving the process to step S51.

Then, in the judgment processing of step S51, the CPU 5 judges whether or not the ultrasound data to be subject to image processing is B-frame data, and when the ultrasound data is B-frame data, the CPU 5 executes routines from step S52, and otherwise moves the process to step S58.

It is noted that since the operation of the routines from step S52 to step S57 is similar as in the second embodiment, description thereof will be omitted.

The CPU 5 determines a display range R' that is currently executed in the processing of step S58.

Then, the CPU 5 obtains an enlargement ratio α by using the numerical values of the display range R and the display range R' in the following processing of step S59. When, for example, the display range R is 9 cm and the display range R' is 4 cm, the enlargement ratio α will be 9/4.

Then, the CPU 5 respectively multiplies the start coordinate S, "A" and the end coordinate E, "B" of an ROI 41 by the obtained enlargement ratio α to calculate the start coordinate ROI_S, "A" and the end coordinate ROI_E, "B" after the enlargement of the ROI 41, thereafter moving the process to step S60.

The operation of the routines from step S60 to step S64 is similar to that from step S18 to step S22 in the second embodiment.

Therefore, performing such processing makes it possible to dispose an ROI 41 in an ultrasonic image based on an enlarged display range R' as shown in Fig. 24 even if the display range R is enlarged into the display range R' while the ROI 41 is being displayed during the execution of a flow mode. Further, the marker 40A indicating the focus position is disposed at the approximate center position of the ROI 41 even if the display range is enlarged.

Thus, even if the operator changes the display range R, the operator can obtain an easy-to-see ultrasound image (blood flow image) of an ROI 41.

Therefore, according to the third embodiment, the configuration that control is performed to automatically adjust the size of an ROI 41 such that the ROI 41 is disposed within the display region of an ultrasound image when the display region (display range R) displayed on the monitor 8 is changed in the ultrasound image during the execution of a flow mode makes it possible to obtain an easy-to-see ultrasound image (blood flow image) of the ROI 41 even if the display range R is enlarged. Other advantages are the same as those of the second embodiment.

By the way, when observation of a subject is performed by using the ultrasound diagnostic apparatus 1, an ultrasound probe 2 to be connected to the ultrasound diagnostic apparatus 1 is selected from a plural kinds of ultrasound probes according to the type of site to be observed. Therefore, every time a different type of ultrasound probe 2 is connected, the focus position needs to be adjusted, which is inconvenient.

Then, in the ultrasound diagnostic apparatus 1 of the present invention, even when a different type of ultrasound probe 2 is used according to the type of site to be observed, it is possible to set the focus position to be suitable for the connected ultrasound probe 2. Such an embodiment will be described later.

### (Fourth Embodiment)

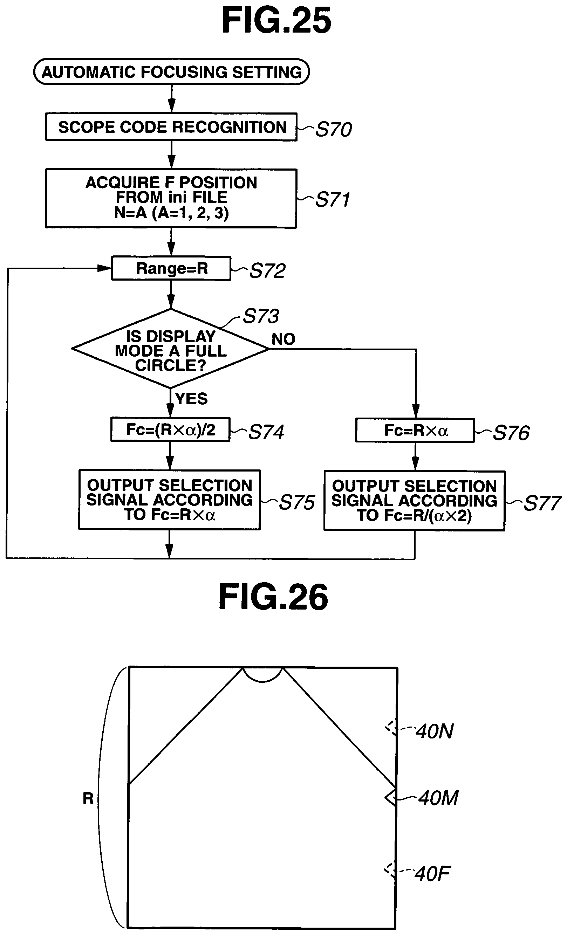

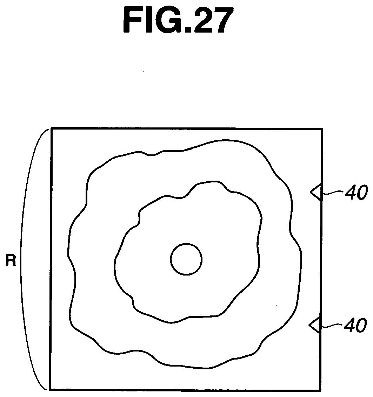

Figs. 25 to 27 relate a fourth embodiment not forming part of the present invention, in which Fig. 25 is a flowchart showing the control processing of a CPU to illustrate the action of an ultrasound diagnostic apparatus relating to the fourth embodiment; Fig. 26 is a screen display diagram showing a state in which the focus position is set for each kind of connected ultrasound probes in the display screen of an ultrasound image of a half circle display mode; and Fig. 27 is a screen display diagram showing a state in which the focus position suitable for a connected ultrasound probe is set in the display screen of an ultrasound image of a full circle display mode.

An ultrasound diagnostic apparatus 1 of the fourth embodiment is similarly configured as the ultrasound diagnostic apparatus 1 of the first embodiment, but differs in the control operation of the CPU 5 constituting control means, and in that user setup data is stored in a storage unit 20.

The user setup data stored in the storage unit 20 includes, for example, a scope code that is assigned to each kind of the ultrasound probe 2, and initial setup information (which is, for example, stored in an "ini" file, etc.) of focus position according to the kind of the ultrasound probe 2, etc.

Then, the CPU 5 reads out the initial setup information based on the ultrasound probe 2 to be connected to a digital beam former 3, and performs the control to change the focus position based on the read-out initial setup information.

Other configurations are the same as those of the first embodiment.

Next, the action of the ultrasound diagnostic apparatus 1 of the fourth embodiment will be described by using Figs. 25 to 27.

Now, suppose that the operator has performed the operation for automatic focusing setup of the ultrasound image displayed in the monitor 8 by use of the ultrasound diagnostic apparatus 1 shown in Fig. 1, or has manipulated a fifth button 35 (for focus presetting) of the keyboard 18 shown in Fig. 4.

In this case, the CPU 5 of Fig. 1 reads out and executes a program for the processing shown in Fig. 25, and stored in the storage unit 20.

The CPU 5 detects a connected ultrasound probe 2 in the processing of step S70, and compares the detection result with the user setup data stored in the storage unit 20, thereby recognizing a scope cord of the connected ultrasound probe 2.

Then, in the following processing of step S71, the CPU 5 acquires a focus position N (indicated by "A" in Fig. 25) that is preset according to the recognized scope code by using the ini file of the initial setup information stored in the storage unit 20.

In this case, the focus position N is represented by "A", and "A" is assigned to three-stage focus positions, that is, 1, 2, and 3 focus positions, for example, for each kind of the ultrasound probe 2. Moreover, the CPU 5 acquires the data of α according to 1, 2, and 3 focus positions of "A" from the ini file of the initial setup information.

The data of α is, for example, 1/4 when "A" is 1, 1/2 when "A" is 2, and 3/4 when "A" is 3.

It is noted that the initial setup information of focus position is not limited to one that is preset for the above-described three stages, and may be increased or decreased as needed.

Thereafter, the CPU 5 determines the display range R in a currently executed mode in the processing of step S72, and moves to the following judgment processing of step S73.

In the judgment processing of step S73, the CPU 5 judges whether or not the currently executed display mode is a full circle display mode in a radial display, and when the display mode is the full circle display mode, the CPU 5 moves the process to step S74, and when the display mode is not the full circle display mode, moves the process to step S76.

Here, first, description will be made on the case in which the display mode is not the full circle display mode, but the half circle display mode.

When the CPU 5 judges that the display mode is not the full circle display mode in the judgment processing of step S73, the CPU 5 recognizes that the display region is changed from the full circle display mode to the half circle display mode, and calculates and sets the focus position Fc at R × α, thereafter moving the process to step S77.

In the processing of step S77, the CPU 5 generates a selection signal that disposes the focus position Fc at R × α, and outputs the signal to the timing generator 23 via the scan controller 22 in the controller 10.

By making the operation thereafter to be performed in the same manner as in the first embodiment, the focus position Fc of the ultrasound image displayed on the monitor 8 is changed.

Therefore, performing such processing makes it possible to dispose a marker 40N indicating a focus position in a shallow area of the upper part of a monitor 8 for example as shown in Fig. 26, when a connected ultrasound probe 2 is for the purpose of observing a subject having a shallow focus position such as a bronchus and a lymph vessel, etc. with a small diameter probe.

Moreover, when the ultrasound probe 2 is for the purpose of observing the inside of a subject having a relatively deep focus position with a large diameter probe, it becomes possible to dispose a marker 40F indicating a focus position in a relatively deep area in the lower part of the monitor 8.

Moreover, when the ultrasound probe 2 is for the purpose of observing a subject at a middle point of the above-described focus positions, it becomes possible to dispose a marker 40M indicating the focus position at a position of 1/2 of display range R or in the vicinity thereof as in the first embodiment.

Next, when the CPU 5 judges that the display mode is the full circle display mode in the judgment processing of step S73, the CPU 5 calculates and sets the focus position Fc at (R × α)/2 or in the vicinity thereof by the processing of step S74, thereafter moving the process to step S75.

The CPU 5 generates a selection signal that disposes the focus position Fc at (R × α)/2 or in the vicinity thereof in the processing of step S75, and outputs the signal to the timing generator 23 via the scan controller 22 in the controller 10.

By making the operation thereafter to be performed in the same manner as in the first embodiment, the focus position Fc of the ultrasound image displayed on the monitor 8 is changed.

Therefore, since as a result of performing such processing, a full circle display mode is executed by radial scanning, the CPU 5 can dispose any of the marker 40N, the marker 40M, and the marker 40F (see Fig. 26) according to the kind of the ultrasound probe 2 even in the full circle display mode as in the case of the half circle display mode.

It is noted that Fig. 27 shows a screen display example in which a diagnostic ultrasound probe 2 is judged to be connected, and a marker 40 indicting the focus position is disposed at each position of 1/4 of the display range R or in the vicinity thereof. In such a case, it is possible to facilitate the observation of the entire ultrasound image in the middle of the focus positions.

Therefore, according to the fourth embodiment, since the CPU 5 can read out the above-described initial setup information based on the ultrasound probe 2 to be connected to a digital beam former 3, and control the focus position to be changed based on the readout initial setup information, it is possible to ease the operation to set the focus position when a different type of ultrasound probes 2 is connected.

### (Fifth Embodiment)

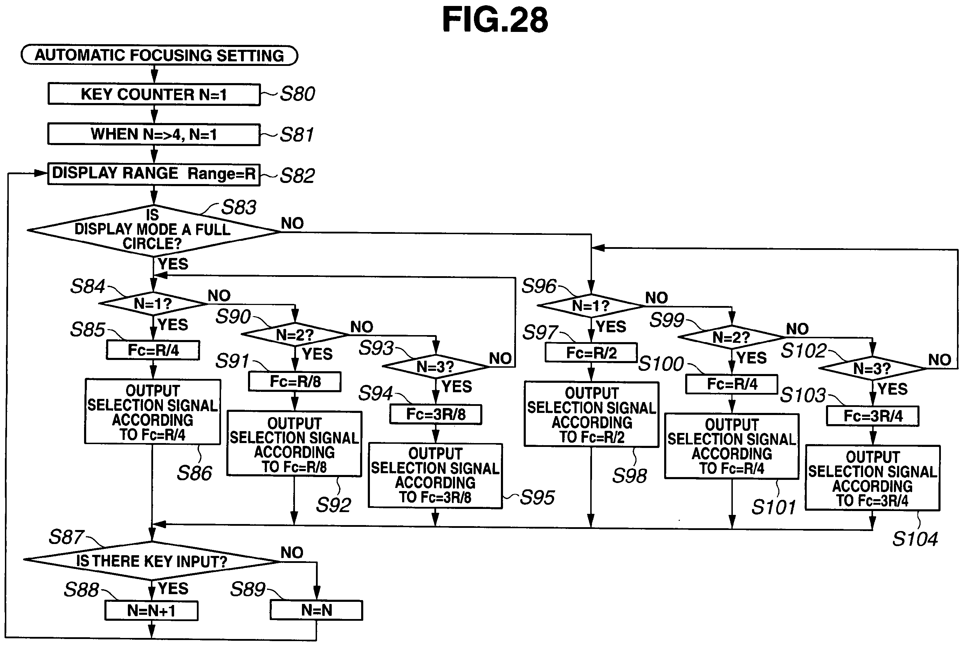

Fig. 28, which relates to a fifth embodiment not forming part of the present invention, is a flowchart showing the flow of the control processing of a CPU to illustrate the action of an ultrasound diagnostic apparatus relating to the fifth embodiment.

The ultrasound diagnostic apparatus 1 of the fifth embodiment is similarly configured as the ultrasound diagnostic apparatus 1 of the first embodiment, but differs in the control operation of the CPU 5 constituting control means.

To be specific, the CPU 5 controls the focus position of an ultrasound image to be set to any of three-stage positions of a near point position, a middle point position, and a far point position, based on the operation by the keyboard 18.

It is noted that in this case, an operator performs toggle operation by using a fifth button 35 of the keyboard 18. The CPU 5 successively sets the focus position of the ultrasound image displayed on the monitor 8 to be at a near point position, a middle point position, or a far point position based on the toggle operation of the fifth button 35 by the operator.

Moreover, the CPU 5 includes a key counter for counting the number of toggle operation by the fifth button 35. That is, the count number N varies in such a way as 1, 2, 3, 1, 2, 3, ...

The other configurations are the same as those of the first embodiment.

Next, the action of the ultrasound diagnostic apparatus 1 of the fifth embodiment will be described with reference to Fig. 28.

Now, suppose that an operator has performed the operation to perform automatic focusing setup of the ultrasound image displayed on the monitor 8 by using the ultrasound diagnostic apparatus 1 shown in Fig. 1, or has manipulated the fifth button 35 of the keyboard 18 shown in Fig. 4.

In this case, the CPU 5 of Fig. 1 reads out and executes a program for the processing shown in Fig. 28 and stored in the storage unit 20.

The CPU 5 lets the counter value N of the key counter not shown be 1 in the processing of step S80, and in the following processing of step S81, sets the count value N to 1 when the count value N becomes not less than 4, thereafter moving the process to step S82.

In the processing of step S82, the CPU 5 determines and sets the display range R in the currently executed mode, and moves to the following judgment processing of step S83.

In the judgment processing of step S83, the CPU 5 judges whether or not the currently executed display mode is a full circle display mode in a radial display, and when the display mode is the full circle display mode, the CPU 5 moves the process to step S84, and when the display mode is not the full circle display mode, moves the processing to step S96.

In the processing of step S84, the CPU 5 judges whether or not the count value N is 1, and when the count value N is 1, the CPU 5 moves the process to step S85, and otherwise moves the process to step S90.

In the processing of step S85, since in this case, the display mode is a full circle display mode, the CPU 5 calculates and sets the focus position Fc at R/4 or in the vicinity thereof, and generates a selection signal that disposes the focus position Fc at R/4 by the following processing of step S86, and outputs the signal to the timing generator 23 via the scan controller 22 in the controller 10, thereby moving the process to step S87. It is noted that performing the processing of step S85 will cause a selection signal that disposes the focus position Fc at a far point position to be outputted.

On the other hand, when the count value N is not 1, the CPU 5 determines whether or not the count value N is 2 in the judgment processing of step S90. When the count value N is 2, the CPU 5 moves the process to step S91, and otherwise moves the process to step S93.

The CPU 5 calculates and sets the focus position Fc at R/8 or in the vicinity thereof in the processing of step S91. Then, the CPU 5 generates a selection signal that disposes the focus position Fc at R/8 or in the vicinity thereof by the following processing of step S91, and outputs the signal to the timing generator 23 via the scan controller 22 in the controller 10, thereafter moving the process to step S87. It is noted that performing the processing of step S91 will cause a selection signal that disposes the focus position Fc at a middle point position to be outputted.

Moreover, when the count value N is not 2, the CPU 5 determines whether or not the count value N is 3 in the judgment processing of step S93. Then, when the count value N is 3, the CPU 5 moves the process to step S94, and otherwise returns the process to step S84.

In the processing of step S94, the CPU 5 calculates and sets the focus position Fc at 3R/8 or in the vicinity thereof. Then, the CPU 5 generates a selection signal that disposes the focus position Fc at 3R/8 or in the vicinity thereof by the following processing of step S91, and outputs the signal to the timing generator 23 via the scan controller 22 in the controller 10, thereafter moving the process to step S87. It is noted that performing the processing of step S94 will cause a selection signal that disposes the focus position Fc at a near point position to be outputted.

Next, when the display mode is judged not to be a full circle display mode in the judgment processing of step S83, the CPU 5 recognizes that the display mode is changed from a full circle display mode to a half circle display mode. Then, the CPU 5 judges whether or not the count value N is 1 in the judgment processing of step S96, and when the count value N is 1, moves the process to step S97 and otherwise moves the process to step S99.

In the processing in step S97, since in this case, the display mode is a half circle display mode, the CPU 5 calculates and sets the focus position Fc at R/2 or in the vicinity thereof then the CPU 5 generates a selection signal that disposes the focus position Fc at R/2 or in the vicinity thereof by the following processing of step S98, and outputs the same to the timing generator 23 via the scan controller 22 in the controller 10, thereafter moving the process to step S87. It is noted that performing the processing of step S97 causes a selection signal that disposes the focus position Fc at a middle point position to be outputted.

On the other hand, when the count value N is not 1, the CPU 5 determines whether or not the count value N is 2 in the judgment processing of step S99. Then, when the count value N is 2, the CPU 5 moves the process to step S100, and otherwise moves the process to step S102.

In the processing of step S100, the CPU 5 calculates and sets the focus position Fc at R/4 or in the vicinity thereof. Then, the CPU 5 generates a selection signal that disposes the focus position Fc at R/4 or in the vicinity thereof by the following processing of step S101, and outputs the same to the timing generator 23 via the scan controller 22 in the controller 10, thereafter moving the process to step S87. It is noted that performing the processing of step S100 causes a selection signal that disposes the focus position at a near point position to be outputted.

Moreover, when the count value N is not 2, the CPU 5 determines whether or not the count value N is 3 in the judgment processing of step S102. Then, when the count value is 3, the CPU 5 moves the process to step S103, and otherwise returns the process to step S96.

In the processing of step S103, the CPU 5 calculates and sets the focus position Fc at 3R/4 or in the vicinity thereof. Then, the CPU 5 generates a selection signal that disposes the focus position Fc at a position of 3R/4 or in the vicinity thereof by the following processing of step S104, and outputs the signal to the timing generator 23 via the scan controller 22 in the controller 10, thereafter moving the process to step S87. It is noted that performing the processing of step S103 causes a selection signal that disposes the focus position Fc at a far point position to be outputted.

Thereafter, the CPU 5 judges whether or not there is a key input by the fifth button 35 of the keyboard 18 in the processing of step S87. Then, when there is a key input, the CPU 5 adds 1 to the count value N in the processing of step S88, then returning the process to step S82, and when there is no key input, keeps the count value N as it is in the processing of step S89, then returning the process to step S82.

As so far described, performing the processing described above makes it possible to successively set the focus position of the ultrasound image displayed on the monitor 8 to any of three-stage positions, a near point position, a middle point position, and a far point position by the operator performing toggle operation of the fifth button 35 of the keyboard 18, even when the display mode is a full circle display mode or a half circle display mode by a radial scanning.

Therefore, according to the fifth embodiment, the operator can change the focus position of the ultrasound image with a simple operation, since the CPU 5 can control the focus position of an ultrasound image to be set to any of three-stage positions of a near point position, a middle point position, and a far point position based on the operation through the keyboard 18.

Although, in the fifth embodiment, description has been made that the focus position of an ultrasound image is changed in three stages of a near point position, a middle point position, and a far point position, this is not restrictive and configuration may be such that more focus positions are preset, and the focus positions are successively set through toggle operation of the fifth button 35 of the keyboard 18.

Moreover, although in the fifth embodiment, description has been made on the case in which a B-mode is executed, the fifth embodiment is of course effective during the execution of a flow mode.

The present application is filed claiming the priority of Japanese Patent Application No. 2008-231244 filed on September 9, 2008.

## Claims

1. An ultrasound diagnostic apparatus (1) configured to transmit ultrasound from an ultrasound transducer (2a) to a subject and display an ultrasound image on a monitor (8), the ultrasound diagnostic apparatus (1) comprising:
transducer driving means (11) configured to transmit ultrasound with a predetermined focus position from the ultrasound transducer (2a);
display region changing means (10) comprising a scan controller (22) and a timing generator (23), the scan controller (22) comprising a digital signal processor (25), a dual port RAM (26) and a scan control section (27), and the timing generator (23) comprising a flash memory (28), a timing generation section (29), and a Tx control section (30), wherein the Tx control section (30) is configured to output to the transducer driving means (11) a control signal for changing transmission timing of the ultrasound transmitted by the transducer driving means (11) based on a timing signal from the timing generation section (29), according to an instruction from the scan control section (27), the display region changing means (10) being configured to change a display region to be displayed on the monitor (8), in an ultrasound image; and
control means (5) configured to change a focus position of the ultrasound by controlling the transducer driving means (11) according to a change result of the display region changing means (10), wherein the scan control section (27) is configured to control the timing generator (23) according to an instruction from the control means (5),
**characterized in that** the control means (5) are configured to
determine a display range R in a currently executed display mode;
judge whether the currently executed display mode is a full circle display mode or a half circle display mode;
generate a selection signal disposing the focus position at R/4 or in the vicinity thereof and output the generated selection signal to the timing generator (23) in case the currently executed display mode is a full circle display mode so that an ultrasound image of a full circle display is displayed in which a marker (40) indicating the focus position is disposed at two points along one side of a display frame and at positions at 1/4 of the display range or in the vicinity thereof, and the focus position is located at the position of the marker (40); and
generate a selection signal disposing the focus position at R/2 or in the vicinity thereof and output the generated selection signal to the timing generator (23) in case the currently executed display mode is a half circle display mode so that an ultrasound image of a half circle display is displayed in which a marker (40) indicating the focus position is disposed at a position of 1/2 of the display range R or in the vicinity thereof, and the focus position is located at the position of the marker (40).

2. The ultrasound diagnostic apparatus (1) according to claim 1, wherein
the control means (5) are configured to change the focus position of the ultrasound image by controlling a transmission timing of timing adjustment means (3) that adjust the transmission timing of the ultrasound in the transducer driving means (11).

3. The ultrasound diagnostic apparatus (1) according to claim 2, further comprising
storage means (20) configured to store transmission timing information according to a display region to be displayed on the monitor (8) in the ultrasound image, wherein
when the display region is changed, the control means (5) are configured to read out the transmission timing information according to the display region after change from the storage means (20), and control the timing adjustment means (3), thereby changing the focus position of the ultrasound image based on the read-out transmission timing information.

4. The ultrasound diagnostic apparatus (1) according to any one of claims 1 to 3, wherein
the display region to be displayed on the monitor (8) in the ultrasound image is the full circle display in which the ultrasound image is radially displayed during radial scanning of the ultrasound transducer (2a), or the half circle display that is a half of the full circle display, or a display region based on a region of interest in the ultrasound image.

5. The ultrasound diagnostic apparatus (1) according to claim 3, wherein
the control means (5) are configured to change a focus position of an ultrasound image of a B-frame, and a focus position of an ultrasound image of a flow frame, respectively during execution of a flow mode.

6. The ultrasound diagnostic apparatus (1) according to claim 5, wherein
when a display region to be displayed on the monitor (8) in an ultrasound image during the execution of a flow mode is changed, the control means (5) are configured to adjust the display size of a region of interest such that the region of interest is disposed within the display region of the ultrasound image.

7. The ultrasound diagnostic apparatus (1) according to claim 3, wherein
the storage means (20) are further configured to store initial setup information of focus position according to a kind of an ultrasound probe having the ultrasound transducer (2a), and
the control means (5) are configured to read out the initial setup information based on the ultrasound probe to be connected to ultrasound transmission/reception means (3) and change the focus position based on the read-out initial setup information.

8. The ultrasound diagnostic apparatus (1) according to any one of claims 2 to 3, further comprising operation means (18) configured to change a focus position of the ultrasound image, wherein
the control means (5) are configured to set the focus position of the ultrasound image to any of three-stage positions of a near point position, a middle point position, and a far point position based on an operation of the operation means (18).

9. The ultrasound diagnostic apparatus (1) according to claim 3, wherein
the storage means (20) includes information of a delay amount according to a sequence position of the ultrasound transducer (2a) as the transmission timing information.

10. A method for controlling a focus position of an ultrasound diagnostic apparatus (1) that transmits ultrasound from an ultrasound transducer (2a) to a subject and displays an ultrasound image on a monitor (8), wherein
the method for controlling a focus position of the ultrasound diagnostic apparatus (1) comprises:
changing a display region to be displayed on the monitor (8) in the ultrasound image; and
changing the focus position of the ultrasound by controlling transducer driving means (11) that drive the ultrasound transducer (2) according to the result of the changing of the display region,
**characterized by**
determining a display range R in a currently executed display mode;
judging whether the currently executed display mode is a full circle display mode or a half circle display mode;
generating a selection signal disposing the focus position at R/4 or in the vicinity thereof and outputting the generated selection signal to a timing generator (23) that is configured to change transmission timing of the ultrasound in case the currently executed display mode is a full circle display mode so that an ultrasound image of a full circle display is displayed in which a marker (40) indicating the focus position is disposed at two points along one side of a display frame and at positions at 1/4 of the display range or in the vicinity thereof, and the focus position is located at the position of the marker (40); and
generating a selection signal disposing the focus position at R/2 or in the vicinity thereof and outputting the generated selection signal to the timing generator (23) in case the currently executed display mode is a half circle display mode so that an ultrasound image of a half circle display is displayed in which a marker (40) indicating the focus position is disposed at a position of 1/2 of the display range R or in the vicinity thereof, and the focus position is located at the position of the marker (40).

11. The method for controlling a focus position of an ultrasound diagnostic apparatus (1) according to claim 10, wherein
a focus position of the ultrasound image is changed by controlling transmission timing of timing adjustment means (3) that adjust a transmission timing of the ultrasound in the transducer driving means (11).

12. The method for controlling a focus position of an ultrasound diagnostic apparatus (1) according to claim 11, wherein
when the display region is changed, transmission timing information according to a display region after change is read out from storage means (20) that store transmission timing information according to a display region to be displayed on the monitor (8) in the ultrasound image, and the timing adjustment means (3) is controlled based on the read-out transmission timing information to change a focus position of the ultrasound image.

13. The method for controlling a focus position of an ultrasound diagnostic apparatus (1) according to any one of claims 10 to 12, wherein
the display region to be displayed on the monitor (8) in the ultrasound image is the full circle display in which the ultrasound image is radially displayed during a radial scanning of the ultrasound transducer (2a), or the half circle display that is half of the full circle display, or a display region based on a region of interest displayed in the ultrasound image.

## Patentansprüche

1. Ultraschalldiagnosegerät (1), das so konfiguriert ist, dass es Ultraschall von einem Ultraschallwandler (2a) an ein Subjekt überträgt und ein Ultraschallbild auf einem Monitor (8) anzeigt, wobei das Ultraschalldiagnosegerät (1) umfasst:
eine Wandler-Antriebseinrichtung (11), die so konfiguriert ist, dass sie Ultraschall mit einer vorgegebenen Fokusposition vom Ultraschallwandler (2a) überträgt;
eine Anzeigebereich-Änderungseinrichtung (10), umfassend einen Abtast-Controller (22) und einen Zeitgeber (23), wobei der Abtast-Controller (22) einen Digitalsignalprozessor (25), ein Doppelport-RAM (26) und einen Abtast-Kontrollabschnitt (27) umfasst, und der Zeitgeber (23) einen Flashspeicher (28), einen Zeitgebungsabschnitt (29) und einen Tx-Steuerabschnitt (30) umfasst, wobei der Tx-Steuerabschnitt (30) so konfiguriert ist, dass er an die Wandler-Antriebseinrichtung (11) ein Steuersignal zur Änderung der Übertragungszeit des von der Wandler-Antriebseinrichtung (11) übertragenen Ultraschalls ausgibt, und zwar auf der Grundlage eines Zeitgebungssignals vom Zeitgebungsabschnitt (29) gemäß einem Befehl vom Abtast-Kontrollabschnitt (27), wobei die Anzeigebereich-Änderungseinrichtung (10) so konfiguriert ist, dass sie einen auf dem Monitor (8) anzuzeigenden Anzeigebereich in einem Ultraschallbild ändert; und
eine Steuereinrichtung (5), die so konfiguriert sind, dass sie eine Fokusposition des Ultraschalls durch Steuerung der Wandler-Antriebseinrichtungs (11) gemäß einem Änderungsergebnis der Anzeigebereich-Änderungseinrichtung (10) ändern, wobei der Abtast-Kontrollabschnitt (27) zur Steuerung des Zeitgebers (23) gemäß einem Befehl von der Steuereinrichtung (5) konfiguriert ist,
**dadurch gekennzeichnet, dass** die Steuereinrichtung (5) konfiguriert ist zur
Bestimmung eines Anzeigebereichs R in einem aktuell ausgeführten Anzeigemodus;
Beurteilung, ob der aktuell ausgeführte Anzeigemodus ein Vollkreisanzeigemodus oder ein Halbkreisanzeigemodus ist;
Erzeugung eines Auswahlsignals, das die Fokusposition bei R/4 oder in der Nähe davon anordnet, und Ausgabe des erzeugten Auswahlsignals an den Zeitgeber (23), im Fall, dass der aktuell ausgeführte Anzeigemodus ein Vollkreisanzeigemodus ist, so dass ein Ultraschallbild einer Vollkreisanzeige angezeigt wird, in der ein die Fokusposition anzeigender Marker (40) an zwei Punkten entlang einer Seite des Anzeigerahmens und an Positionen von 1/4 des Anzeigebereichs oder in der Nähe davon angeordnet ist, und die Fokusposition sich an der Position des Markers (40) befindet; und
Erzeugung eines Auswahlsignals, das die Fokusposition bei R/2 oder in der Nähe davon anordnet, und Ausgabe des erzeugten Auswahlsignals an den Zeitgeber (23), im Fall, dass der aktuell ausgeführt Anzeigemodus ein Halbkreisanzeigemodus ist, so dass ein Ultraschallbild einer Halbkreisanzeige angezeigt wird, in der ein die Fokusposition anzeigender Marker (40) an einer Position von 1/2 des Anzeigebereichs R oder in der Nähe davon angeordnet wird, und die Fokusposition sich an der Position des Markers (40) befindet.

2. Ultraschalldiagnosegerät (1) nach Anspruch 1, wobei
die Steuereinrichtung (5) so konfiguriert ist, dass sie die Fokusposition des Ultraschallbildes ändert, indem sie eine Übertragungszeit der Zeiteinstellungseinrichtung (3) steuert, die den Übertragungszeitablauf des Ultraschalls in der Wandler-Antriebseinrichtung (11) einstellt.

3. Ultraschalldiagnosegerät (1) nach Anspruch 2, weiterhin umfassend
eine Speichereinrichtung (20), die so konfiguriert ist, dass sie Übertragungszeitinformation gemäß einem auf dem Monitor (8) anzuzeigenden Anzeigebereich im Ultraschallbild speichert, wobei
wenn sich der Anzeigebereich ändert, die Steuereinrichtung (5) so konfiguriert ist, dass sie die Übertragungszeitinformation gemäß dem Anzeigebereich nach der Änderung aus der Speichereinrichtung (20) ausliest, und dass sie die Zeiteinstellungseinrichtung (3) steuert und damit die Fokusposition des Ultraschallbildes auf der Grundlage der ausgelesenen Übertragungszeitinformation ändert.

4. Ultraschalldiagnosegerät (1) nach einem der Ansprüche 1 bis 3, wobei
der auf dem Monitor (8) anzuzeigende Anzeigebereich im Ultraschallbild die Vollkreisanzeige ist, in der das Ultraschallbild während einer radialen Abtastung des Ultraschallwandlers (2a) radial angezeigt wird, bzw. die Halbkreisanzeige ist, die eine Hälfte der Vollkreisanzeige ist, bzw. ein Anzeigebereich ist, der auf einem interessierenden Bereich im Ultraschallbild basiert.

5. Ultraschalldiagnosegerät (1) nach Anspruch 3, wobei
die Steuereinrichtung (5) so konfiguriert ist, dass sie eine Fokusposition eines Ultraschallbildes eines B-Bildes und eine Fokusposition eines Ultraschallbildes eines Flussbildes jeweils während der Ausführung eines Flussmodus ändert.

6. Ultraschalldiagnosegerät (1) nach Anspruch 5, wobei
wenn sich ein auf dem Monitor (8) anzuzeigender Anzeigebereich in einem Ultraschallbild während der Ausführung eines Flussmodus ändert, die Steuereinrichtung (5) so konfiguriert ist, dass sie die Anzeigegröße eines interessierenden Bereichs einstellt, so dass der interessierende Bereich im Anzeigebereich des Ultraschallbildes angeordnet ist.

7. Ultraschalldiagnosegerät (1) nach Anspruch 3, wobei
die Speichereinrichtung (20) weiterhin so konfiguriert ist, dass sie Ersteinrichtungsinformation einer Fokusposition gemäß einer Art von Ultraschallsonde speichert, die den Ultraschallwandler (2a) aufweist, und
die Steuereinrichtung so konfiguriert ist, dass sie die Ersteinrichtungsinformation auf der Grundlage der an der Ultraschallübertragungs-/empfangseinrichtung (3) anzuschließenden Ultraschallsonde ausliest und die Fokusposition auf der Grundlage der ausgelesenen Ersteinrichtungsinformation ändert.

8. Ultraschalldiagnosegerät (1) nach einem der Ansprüche 2 bis 3, weiterhin umfassend eine Betätigungseinrichtung (18), die so konfiguriert ist, dass sie eine Fokusposition des Ultraschallbildes ändert, wobei
die Steuereinrichtung (5) so konfiguriert ist, dass sie die Fokusposition des Ultraschallbildes auf eine von dreistufigen Positionen einer Nahpunktposition, einer Mittelpunktposition und einer Fernpunktposition auf der Grundlage einer Betätigung der Betätigungseinrichtung (18) einstellt.

9. Ultraschalldiagnosegerät (1) nach Anspruch 3, wobei
die Speichereinrichtung (20) Information eines Verzögerungsbetrags gemäß einer Sequenzposition des Ultraschallwandlers (2a) als die Übertragungszeitinformation enthält.

10. Verfahren zur Steuerung einer Fokusposition eines Ultraschalldiagnosegeräts (1), das Ultraschall von einem Ultraschallwandler (2a) an ein Subjekt überträgt und ein Ultraschallbild auf einem Monitor (8) anzeigt, wobei
das Verfahren zur Steuerung einer Fokusposition des Ultraschalldiagnosegeräts (1) umfasst:
Ändern eines auf dem Monitor (8) anzuzeigenden Anzeigebereichs im Ultraschallbild; und
Ändern der Fokusposition des Ultraschalls durch Steuerung einer Wandler-Antriebseinrichtungen (11), die den Ultraschallwandler (2) gemäß dem Ergebnis der Änderung des Anzeigebereichs antreiben,
**gekennzeichnet durch**
Bestimmen eines Anzeigebereichs R in einem aktuell ausgeführten Anzeigemodus;
Beurteilen, ob der aktuell ausgeführte Anzeigemodus ein Vollkreisanzeigemodus oder ein Halbkreisanzeigemodus ist;
Erzeugen eines Auswahlsignals, das die Fokusposition bei R/4 oder in der Nähe davon anordnet, und Ausgeben des erzeugten Auswahlsignals an einen Zeitgeber (23), der so konfiguriert ist, dass er die Übertragungszeit des Ultraschalls ändert, im Fall, dass der aktuell ausgeführte Anzeigemodus ein Vollkreisanzeigemodus ist, so dass ein Ultraschallbild einer Vollkreisanzeige angezeigt wird, in der ein Marker (40), der die Fokusposition anzeigt, an zwei Punkten entlang einer Seite des Anzeigerahmens und an Positionen von 1/4 des Anzeigebereichs oder in der Nähe davon angeordnet wird, und die Fokusposition sich an der Position des Markers (40) befindet; und
Erzeugen eines Auswahlsignals, das die Fokusposition bei R/2 oder in der Nähe davon anordnet, und Ausgeben des erzeugten Auswahlsignals an den Zeitgeber (23), im Fall, dass der aktuell ausgeführte Anzeigemodus ein Halbkreisanzeigemodus ist, so dass ein Ultraschallbild einer Halbkreisanzeige angezeigt wird, in der ein Marker (40), der die Fokusposition anzeigt, an einer Position von 1/2 des Anzeigebereichs R oder in der Nähe davon angeordnet wird, und die Fokusposition sich an der Position des Markers (40) befindet.

11. Verfahren zur Steuerung einer Fokusposition eines Ultraschalldiagnosegeräts (10) nach Anspruch 10, wobei
eine Fokusposition des Ultraschallbildes durch Steuerung der Übertragungszeit einer Zeiteinstellungseinrichtung (3) geändert wird, die eine Übertragungzeit des Ultraschalls in der Wandler-Antriebseinrichtung (11) einstellt.

12. Verfahren zur Steuerung einer Fokusposition eines Ultraschalldiagnosegeräts (1) nach Anspruch 11, wobei
wenn sich der Anzeigebereich ändert, die Übertragungszeitinformation gemäß einem Anzeigebereich nach der Änderung aus einer Speichereinrichtung (20) ausgelesen wird, die die Übertragungszeitinformation gemäß einem auf dem Monitor (8) anzuzeigenden Anzeigebereich im Ultraschallbild speichert, und die Zeiteinstellungseinrichtung (3) auf der Grundlage der ausgelesenen Übertragungszeitinformation gesteuert wird, um eine Fokusposition des Ultraschallbildes zu ändern.

13. Verfahren zur Steuerung einer Fokusposition eines Ultraschalldiagnosegeräts (1) nach einem der Ansprüche 10 bis 12, wobei
der auf dem Monitor (8) anzuzeigende Anzeigebereich im Ultraschallbild die Vollkreisanzeige ist, in der das Ultraschallbild während einer radialen Abtastung des Ultraschallwandlers (2a) radial angezeigt wird, bzw. die Halbkreisanzeige ist, die die Hälfte der Vollkreisanzeige beträgt, bzw. ein Anzeigebereich ist, der auf einem im Ultraschallbild angezeigten interessierenden Bereich basiert.

## Revendications

1. Appareil de diagnostic à ultrasons (1) configuré pour transmettre des ultrasons en provenance d'un transducteur à ultrasons (2a) vers un sujet et afficher une image ultrasonique sur un dispositif de surveillance (8), l'appareil de diagnostic à ultrasons (1) comprenant :
des moyens de pilotage de transducteur (11) configurés pour transmettre des ultrasons avec une position de focalisation prédéterminée à partir du transducteur à ultrasons (2a) ;
des moyens de modification de région d'affichage (10) comprenant un dispositif de commande de balayage (22) et un générateur de temporisation (23), le dispositif de commande de balayage (22) comprenant un processeur de signal numérique (25), une RAM à double port (26) et une section de commande de balayage (27), et le générateur de temporisation (23) comprenant une mémoire flash (28), une section de génération de temporisation (29) et une section de commande de transmission, Tx, (30), la section de commande Tx (30) étant configurée pour délivrer aux moyens de pilotage de transducteur (11) un signal de commande pour modifier la temporisation de transmission des ultrasons transmis par les moyens de pilotage de transducteur (11) sur la base d'un signal de temporisation en provenance de la section de génération de temporisation (29), selon une instruction en provenance de la section de commande de balayage (27), les moyens de modification de région d'affichage (10) étant configurés pour modifier une région d'affichage à afficher sur le dispositif de surveillance (8), dans une image ultrasonique ; et
des moyens de commande (5) configurés pour modifier une position de focalisation des ultrasons par commande des moyens de pilotage de transducteur (11) selon un résultat de changement des moyens de modification de région d'affichage (10), la section de commande de balayage (27) étant configurée pour commander le générateur de temporisation (23) selon une instruction en provenance des moyens de commande (5),
**caractérisé par le fait que** les moyens de commande (5) sont configurés pour déterminer une plage d'affichage R dans un mode d'affichage actuellement exécuté ;
déterminer si le mode d'affichage actuellement exécuté est un mode d'affichage en cercle complet ou un mode d'affichage en demi-cercle ;
générer un signal de sélection disposant la position de focalisation au niveau de R/4 ou au voisinage de celui-ci et délivrer le signal de sélection généré au générateur de temporisation (23) dans le cas où le mode d'affichage actuellement exécuté est un mode d'affichage en cercle complet de telle sorte qu'une image ultrasonique d'un affichage en cercle complet est affichée dans laquelle un marqueur (40) indiquant la position de focalisation est disposé au niveau de deux points le long d'un côté d'une trame d'affichage et au niveau de positions à 1/4 de la plage d'affichage ou au voisinage de celles-ci, et la position de focalisation est située au niveau de la position du marqueur (40) ; et
générer un signal de sélection disposant la position de focalisation au niveau de R/2 ou au voisinage de celui-ci et délivrer le signal de sélection généré au générateur de temporisation (23) dans le cas où le mode d'affichage actuellement exécuté est un mode d'affichage en demi-cercle de telle sorte qu'une image ultrasonique d'un affichage en demi-cercle est affichée dans laquelle un marqueur (40) indiquant la position de focalisation est disposé au niveau d'une position de 1/2 de la plage d'affichage R ou au voisinage de celle-ci, et la position de focalisation est située au niveau de la position du marqueur (40).

2. Appareil de diagnostic à ultrasons (1) selon la revendication 1, dans lequel les moyens de commande (5) sont configurés pour modifier la position de focalisation de l'image ultrasonique par commande d'une temporisation de transmission de moyens de réglage de temporisation (3) qui règlent la temporisation de transmission des ultrasons dans les moyens de pilotage de transducteur (11).

3. Appareil de diagnostic à ultrasons (1) selon la revendication 2, comprenant en outre
des moyens de stockage (20) configurés pour stocker des informations de temporisation de transmission selon une région d'affichage à afficher sur le dispositif de surveillance (8) dans l'image ultrasonique, dans lequel
lorsque la région d'affichage est modifiée, les moyens de commande (5) sont configurés pour lire les informations de temporisation de transmission selon la région d'affichage après modification à partir des moyens de stockage (20), et commander les moyens de réglage de temporisation (3), modifiant ainsi la position de focalisation de l'image ultrasonique sur la base des informations de temporisation de transmission lues.

4. Appareil de diagnostic à ultrasons (1) selon l'une quelconque des revendications 1 à 3, dans lequel
la région d'affichage à afficher sur le dispositif de surveillance (8) dans l'image ultrasonique est l'affichage en cercle complet dans lequel l'image ultrasonique est affichée de manière radiale durant un balayage radial du transducteur à ultrasons (2a), ou l'affichage en demi-cercle qui est une moitié de l'affichage en cercle complet, ou une région d'affichage basée sur une région d'intérêt dans l'image ultrasonique.

5. Appareil de diagnostic à ultrasons (1) selon la revendication 3, dans lequel les moyens de commande (5) sont configurés pour modifier une position de focalisation d'une image ultrasonique d'une trame B, et une position de focalisation d'une image ultrasonique d'une trame de flux, respectivement durant l'exécution d'un mode de flux.

6. Appareil de diagnostic à ultrasons (1) selon la revendication 5, dans lequel lorsqu'une région d'affichage à afficher sur le dispositif de surveillance (8) dans une image ultrasonique durant l'exécution d'un mode de flux est modifiée, les moyens de commande (5) sont configurés pour régler la taille d'affichage d'une région d'intérêt de telle sorte que la région d'intérêt est disposée à l'intérieur de la région d'affichage de l'image ultrasonique.

7. Appareil de diagnostic à ultrasons (1) selon la revendication 3, dans lequel les moyens de stockage (20) sont en outre configurés pour stocker des informations de configuration initiale de position de focalisation selon un type d'une sonde ultrasonore ayant le transducteur à ultrasons (2a), et
les moyens de commande (5) sont configurés pour lire les informations de configuration initiale sur la base de la sonde ultrasonore à relier à des moyens de transmission/réception d'ultrasons (3) et modifier la position de focalisation sur la base des informations de configuration initiale lues.

8. Appareil de diagnostic à ultrasons (1) selon l'une quelconque des revendications 2 à 3, comprenant en outre des moyens d'opération (18) configurés pour modifier une position de focalisation de l'image ultrasonique, dans lequel les moyens de commande (5) sont configurés pour régler la position de focalisation de l'image ultrasonique à l'une quelconque de positions à trois étages parmi une position de point proche, une position de point milieu et d'une position de point éloigné sur la base d'une opération des moyens d'opération (18).

9. Appareil de diagnostic à ultrasons (1) selon la revendication 3, dans lequel les moyens de stockage (20) comprennent des informations d'une quantité de retard selon une position de séquence du transducteur à ultrasons (2a) en tant qu'informations de temporisation de transmission.

10. Procédé pour commander une position de focalisation d'un appareil de diagnostic à ultrasons (1) qui transmet des ultrasons en provenance d'un transducteur à ultrasons (2a) vers un sujet et affiche une image ultrasonique sur un dispositif de surveillance (8), dans lequel
le procédé pour commander une position de focalisation de l'appareil de diagnostic à ultrasons (1) comprend :
la modification d'une région d'affichage à afficher sur le dispositif de surveillance (8) dans l'image ultrasonique ; et
la modification de la position de focalisation des ultrasons par commande de moyens de pilotage de transducteur (11) qui pilotent le transducteur à ultrasons (2) selon le résultat de la modification de la région d'affichage,
**caractérisé par**
la détermination d'une plage d'affichage R dans un mode d'affichage actuellement exécuté ;
la détermination du point de savoir si le mode d'affichage actuellement exécuté est un mode d'affichage en cercle complet ou un mode d'affichage en demi-cercle ;
la génération d'un signal de sélection disposant la position de focalisation au niveau de R/4 ou au voisinage de celui-ci et la délivrance du signal de sélection généré à un générateur de temporisation (23) qui est configuré pour modifier la temporisation de transmission des ultrasons dans le cas où le mode d'affichage actuellement exécuté est un mode d'affichage en cercle complet de telle sorte qu'une image ultrasonique d'un affichage en cercle complet est affichée dans laquelle un marqueur (40) indiquant la position de focalisation est disposé au niveau de deux points le long d'un côté d'une trame d'affichage et au niveau de positions à 1/4 de la plage d'affichage ou au voisinage de celles-ci, et la position de focalisation est située au niveau de la position du marqueur (40) ; et
la génération d'un signal de sélection disposant la position de focalisation au niveau de R/2 ou au voisinage de celui-ci et la délivrance du signal de sélection généré au générateur de temporisation (23) dans le cas où le mode d'affichage actuellement exécuté est un mode d'affichage en demi-cercle de telle sorte qu'une image ultrasonique d'un affichage en demi-cercle est affichée dans laquelle un marqueur (40) indiquant la position de focalisation est disposé au niveau d'une position de 1/2 de la plage d'affichage R ou au voisinage de celle-ci, et la position de focalisation est située au niveau de la position du marqueur (40).

11. Procédé pour commander une position de focalisation d'un appareil de diagnostic à ultrasons (1) selon la revendication 10, dans lequel une position de focalisation de l'image ultrasonique est modifiée par commande d'une temporisation de transmission de moyens de réglage de temporisation (3) qui règlent une temporisation de transmission des ultrasons dans les moyens de pilotage de transducteur (11).

12. Procédé pour commander une position de focalisation d'un appareil de diagnostic à ultrasons (1) selon la revendication 11, dans lequel
lorsque la région d'affichage est modifiée, des informations de temporisation de transmission selon une région d'affichage après modification sont lues à partir de moyens de stockage (20) qui stockent des informations de temporisation de transmission selon une région d'affichage à afficher sur le dispositif de surveillance (8) dans l'image ultrasonique, et les moyens de réglage de temporisation (3) sont commandés sur la base des informations de temporisation de transmission lues pour modifier une position de focalisation de l'image ultrasonique.

13. Procédé pour commander une position de focalisation d'un appareil de diagnostic à ultrasons (1) selon l'une quelconque des revendications 10 à 12, dans lequel
la région d'affichage à afficher sur le dispositif de surveillance (8) dans l'image ultrasonique est l'affichage en cercle complet dans lequel l'image ultrasonique est affichée de manière radiale durant un balayage radial du transducteur à ultrasons (2a), ou l'affichage en demi-cercle qui est une moitié de l'affichage en cercle complet, ou une région d'affichage basée sur une région d'intérêt affichée dans l'image ultrasonique.
